# EUROPEAN PATENT SPECIFICATION

(11) **EP 4 254 416 B1**
(45) Date of publication and mention of the grant of the patent: **05.03.2025**
(21) Application number: 22166355.2
(22) Date of filing: 01.04.2022
(51) Int. Cl.: G11C 13/00, C12Q 1/686

(54) **A DEVICE AND A METHOD FOR RECORDING DATA IN NUCLEIC ACIDS**
VORRICHTUNG UND VERFAHREN ZUM AUFZEICHNEN VON DATEN IN NUKLEINSÄUREN
DISPOSITIF ET PROCÉDÉ D'ENREGISTREMENT DE DONNÉES DANS DES ACIDES NUCLÉIQUES

(43) Date of publication of application: 04.10.2023
(73) Proprietor: BioSistemika d.o.o., 1000 Ljubijana (SI)
(72) Inventor: Cepin, Urska, 1292 Ig (SI); Karcnik, Tomaz, 1000 Ljubljana (SI); Kokalj, Tadej, 1236 Trzin (SI); Luzar, Rok, 1234 Menges (SI); Prevorsek, Zala, 4220 Skofja Loka (SI); Stare, Tjasa, 1210 Ljubljana (SI); Solar, Borut, 4209 Zabnica (SI); Zupancic, Luka, 1000 Ljubljana (SI)
(74) Representative: Zacco GmbH

(56) References cited:
- WO-A1-2021/064120
- US-A1- 2010 225 685
- US-A1- 2016 121 280
- US-A1- 2019 362 814
- US-A1- 2020 185 057
- US-A1- 2022 025 428

## Description

### TECHNICAL FIELD

The device and the method according to the present disclosure relate to the field of data recording devices and methods. More specifically, the device and the method disclosed herein relate to recording data in nucleic acids.

### BACKGROUND

In the recent years we have seen an increase in research and development of devices and methods for recording data in nucleic acids for the purpose of storing data in nucleic acids.

Base-to-base nucleic acid synthesis and recording data in nucleotide sequences of nucleic acids, which are synthesised in a base-to-base manner, are described in many documents. Base-to-base nucleic acid synthesis is a costly, slow method, as a separate biochemical reaction has to be performed for an addition and binding of every single nucleotide base. Consequently, recording data in the nucleotide sequences by synthesising nucleic acids in the base-to-base manner, is also costly and slow.

Therefore, new methods of recording data in nucleic acids have been described recently, wherein data is represented in a block-by-block manner, so that basic building blocks, which may be used to represent the data, are nucleic acids, such as: oligonucleotides, DNA fragments, or any other similar nucleic acids which comprise a plurality of nucleotide bases and may be used to represent data. The data may therefore be represented in the block-by-block manner wherein the basic blocks are nucleic acid components such as oligonucleotides and other similar nucleic acids.

As custom precast permutations of oligonucleotides and other nucleic acids, which may be used as the basic building blocks to represent and record data in nucleic acids, are available on the market and may easily be purchased, and the price of such custom precast permutations makes recording data in nucleic acids in the block-by-block manner more accessible, recording data in nucleic acids in the block-by-block manner is becoming more viable than recording data by synthesizing nucleic acids in the base-to-base manner. A set comprising permutations of nucleic acid components, such as oligonucleotides, may therefore be used as a starting nucleic acid library of 'blocks' from which data may be represented.

Data may therefore be represented and recorded in the block-by-block manner wherein the basic building blocks to represent data are nucleic acid components that comprise two or more nucleotide bases, such as oligonucleotides, DNA fragments, plasmids and other similar nucleic acids which may be used to represent the data. When a set of permutations of nucleic acid components is generated or purchased, the set may comprise all permutations and it would be desirable to select a subset of nucleic acid components from the set so that the subset of nucleic acid components represents data, because not all permutations in the set may be needed for representing specific data.

When the set of permutations of nucleic acid components is generated or purchased, each of the permutations is suspended in a liquid at a known concentration, and a subset of nucleic acid components may be selected from the set so that nucleic acid components in the subset represent data. In the next step, one encounters a technical problem of how to position a plurality of nucleic acid components from the subset together in one location, so that they are located next to each other and apart from other non-selected permutations of nucleic acid components from the set. In some cases, it may also be desirable to add binding reagents to the plurality of nucleic acid components in the subset, so that the plurality of nucleic acid components, which were selected to represent data, may assemble and bind together due to the presence of the binding reagent.

However, only a few of publicly available documents describe devices for efficient and cost-effective positioning of the plurality of nucleic acid components, which may be selected from the set of nucleic acid components, together in one location and apart from any other non-selected nucleic acid components.

Some of the biggest problems of developing devices used for recording data in nucleic acids are: speed at which the plurality of nucleic acid components may be positioned in the one location; high reaction volumes that result in a high cost of nucleic acid components and binding reagents used for assembling and binding the nucleic acid components together, evaporation of the binding reagents and liquids in which the components are suspended, speed of assembling and binding of the plurality the nucleic acid components together so that final nucleic acid molecules, that represent data may be produced, contamination risk, amount of plastic consumables used, and other. By using the term 'the plurality of nucleic acid components' it is understood to those skilled in the art, that in many cases, the plurality comprising eight (8) or more nucleic acid components may be used to record data in nucleic acids. To overcome the burdens of manual labour during the steps of positioning the plurality of nucleic acid components, which may represent data, together in the one location, it would be desirable to develop a novel device, which would minimise the time used for positioning the plurality of nucleic acid components together in the one location. To overcome high costs of using high reaction volumes in which the nucleic acid components and the binding reagents are located, it would be desirable to develop a device that would minimise the reaction volumes and at the same time provide a correct concentration of the nucleic acid components and binging reagents in said reaction volumes. By minimising the reaction volumes, costs of recording data in nucleic acids may be drastically lowered. The binding reagents, which are used for assembling and binding of the plurality of nucleic acid components together, are known to be expensive. Therefore, it would be desirable to use as little as possible of such cost-burdening binding reagents within any device used for recording data in nucleic acids. As the binding reagents may also evaporate at a fast pace, it would be desirable to have a device which minimises evaporation of said binding reagents. It is understood that it would also be beneficial to minimise the use of nucleic acid components. Overall, it would be desirable to develop a novel device for recording data in nucleic acids, which would minimise the time used for positioning the plurality of nucleic acid components together in the one location, minimise the reaction volumes and consequently minimise the use of binding reagents and nucleic acid components, minimise evaporation of the binding reagents, minimise contamination risk, minimise the amount of plastic consumables and in this way, minimise an overall cost of the process.

Patent US2020/0185057 describes a device having a printer module and an incubator module. The printer module comprises a plurality of micro-fluid dispensers which dispense droplets containing selected DNA components vertically onto a desired coordinate on the webbing, said webbing which includes a substrate to physically link the plurality of components. The webbing moves through the device collecting the selected DNA of components. Droplets with DNA components are dispensed in drops into individual reaction compartments, and components are later ligated in the incubation module to form identifiers (e.g. longer DNA strings). US 2016/0121280 Al discloses a substance mixing apparatus including two or more flow paths in which orifices, from which a fluid that flows therethrough is externally discharged, are formed, oscillation devices that form droplets of the fluid discharged from each of the orifices by oscillating at least the orifice part of the flow paths at a predetermined oscillation frequency and discharge the droplets, and means for causing the droplets discharged from the orifices of the flow paths to collide with one another.

The invention is defined by the independent claims. Embodiments are given by the dependent claims. A device and a method according to the present disclosure enable a plurality of selected nucleic acid components, which represent data, to be positioned together in one location in a one drop whilst the one drop is in-flight, and before the one drop lands on a drop collecting element. In this way, all selected nucleic acid components in the plurality of nucleic acid components, which represent data, are collected by and located in the one drop before the one drop lands on the drop collecting element. Such a device enables a fast and an efficient positioning of the selected plurality of nucleic acid components, which represent data, in the one location in the one drop. According to at least one preferred example embodiment of the device and the method described herein, the device comprises a plurality of liquid dispensers being configured to dispense drops. A carrier drop is dispensed and sequentially collides and merges with drops that aggregately include the plurality of nucleic acid components, which represent data, so that the plurality of nucleic acid components, which represent data, is located in the carrier drop during flight. A drop collecting element is being configured to collect the carrier drop, which includes the plurality of selected nucleic acid components at the time of landing on the drop collecting element. The plurality of liquid dispensers is configured so that the carrier drop is dispensed and sequentially collides with at least all of the drops that aggregately include a subset of selected nucleic acid components selected from a set of nucleic acid components to represent data, so that all of the nucleic acid components from the subset are collected by and (physically) positioned in the carrier drop before the carrier drop lands on the drop collecting element. In this manner, all of the selected nucleic acid components in the subset, which represent data, are located and physically positioned in the carrier drop before the carrier drop lands on the drop collecting element.

According to at least some example embodiments of the device and the method disclosed herein, at least one liquid dispenser may be configured to dispense drops that include binding reagents suspended in liquid. The binding reagents may aid a biochemical reaction between nucleic acid components, and may cause the nucleic acid components to assemble and bind together into final nucleic acid molecules. In some cases, the binding reagents may also aid a multiplication of the final nucleic acid molecules. In at least some example embodiments of the device and the method disclosed herein, the binding reagents may be included in the carrier drop at a time at which the carrier drop is dispensed, or the binding reagents may be included in a drop that collides with the carrier drop during flight, so that the binding reagents may also be collected by and located in the carrier drop before the carrier drop lands on the drop collecting element. In this way, using at least some example embodiments of the devices and methods disclosed hereof, the subset of nucleic acid components selected to represent data, and the binding reagents, which aid the binding of the nucleic acid components together (and in some cases also the multiplication), may be positioned in the one location in the one drop, which is the carrier drop.

Carrier drops may be dispensed in a stream, which may comprise a first carrier drop, a second carrier drop, and at least a third carrier drop. The plurality of liquid dispensers may be configured so that the first carrier drop may collide and merge with at least drops that aggregately include a first subset of selected nucleic acid components, the second carrier drop may collide and merge with drops that aggregately include a second subset of selected nucleic acid components, the third carrier drop may collide and merge with at least drops that aggregately include components from a third subset of selected nucleic acid components, and so on, until all of the carrier drops in the stream collide with at least some drops that include at least some selected nucleic acid components that represent data, and all of the carrier drops in the stream land on the drop collecting element. In this way, a plurality of distinct carrier drops may be produced in-flight, wherein each distinct carrier drop in the plurality of distinct carrier drops includes all selected nucleic acid components from any one subset of selected nucleic acid components that represents data.

In at least some example embodiments of the device and the method of the present disclosure, the plurality of liquid dispensers is configured to dispense drops of a first liquid, and the drop collecting element may be covered or filled with a second liquid, and the first liquid and the second liquid are immiscible liquids, which do not mix together, so that the carrier drops made of the first liquid are kept intact, are protected from evaporation, and do not merge with each other after landing in the second liquid on the drop collecting element, because the first liquid and the second liquid are immiscible. The second liquid, which is applied on or in the drop collecting element, surrounds or forms a coating surrounding the carrier drops made of the first liquid, immediately after said carrier drops land. Because the second liquid surrounds or forms the coating surrounding the carrier drops after landing, the carrier drops may be protected from evaporation.

For the purpose of avoidance of doubt, the term 'distinct' shall herein mean 'one, which is not any other in the plurality'.

None of publicly mentioned technical solutions relate to a novel device for a rapid in-flight positioning of the plurality of nucleic acid components, which are selected to represent data, in one location within one drop, before the one drop lands. None of the publicly available documents describe a device that would preferably enable a rapid production of the plurality of distinct carrier drops, wherein each of the distinct carrier drops in the plurality of distinct carrier drops includes one subset of nucleic acid components, which represent data, and the binding reagents, which are used for assembling and binding the subset of nucleic acid components together.

The device and the method according to the present disclosure aim to solve a problem relating to positioning a plurality of nucleic acid components, which are selected to represent data, together in one location in a single drop, in-flight, before the single drop lands on the drop collecting element. The advantageous effects of the device and the method according to the present disclosure include a rapid high-throughput production of various combinatorially created distinct carrier drops (e.g. the plurality of distinct carrier drops) that may include any selected combination and number (e.g. subset) of nucleic acid components, which represent data, and a minimal amount of expensive binding reagents, before said carrier drops land on the drop collecting element, which may include a liquid that surrounds the carrier drops after landing, so that said carrier drops are protected from evaporation and do not merge amongst each other after landing.

The device and the method described herein therefore enables collection of the plurality of distinct carrier drops at a same location in the liquid applied in/on the drop collecting element, and no electro-mechanical parts of the device (except for elements that generate drops), need to be physically moved to another location in order to collect the plurality of distinct carrier drops on the drop collecting element. The device and the method may therefore also be less prone to slight mechanical movements of the drop collecting element, as it may be established by using an appropriate set of liquids, which may be immiscible, that the plurality of distinct carrier drops may not merge amongst each other after landing on the drop collecting element, therefore minimising the risk of a cross-contamination of binding reagents, components and the final reaction mixture between the carrier drops after landing. In this way, the plurality of distinct carrier drops may remain intact after landing on the drop collecting element, and are protected from evaporation. It is also important to note that the device and method according to the present disclosure minimise the use of plastic consumables.

### SUMMARY

Devices and methods according to the present disclosure relate to a device and a method for recording data in nucleic acids. The technical problem is solved by the device for recording data in nucleic acids (hereinafter: the device) according to claim 1 and by the method for recording data in nucleic acids (hereinafter: the method) according to claim 15 Further preferred embodiments are provided in the dependent claims.

Terms such as 'a', 'an', and 'the' are not intended to refer to only a singular entity but include the general class of which specific example may be used for illustration. The terminology herein used to describe specific embodiments of the device and the method according to the present disclosure, but their usage does not delimit the invention, except as outlined in the claims.

For the purpose of this disclosure, certain definitions of the following terms shall be used throughout the document:
**array:** the term 'array' is used to refer to a plurality of liquid dispensers arranged in an ordered manner, such as a linear array, a circular array, a mesh array or any other suitable type of array. The array comprises two or more liquid dispensers.
**liquid dispenser (pl. liquid dispensers):** the term 'liquid dispenser' or 'dispenser' is used herein to refer to any liquid dispensing device capable of rapid production of micro-fluid drops with a volume in a range between one (1) picoliter to a hundred (100) nanoliters. The liquid dispenser may be capable of producing from one (1) up to a hundred thousand (100000) drops per second. All drops are dispensed by using liquid dispensers in the device.
**carrier drop (pl. carrier drops):** the term 'carrier drop' or 'carrier drops' is used to refer to a drop in which all components from a subset of components that represents data may be positioned before the drop lands. In at least some example embodiments, the carrier drop may contain binding reagents which may aid a biochemical reaction between the components located in the carrier drop.
**component (pl. components):** shall be considered as a nucleic acid molecule which is capable of representing data. Components comprise a data segment and at least one gluing segment. The data segment of the component represents data, the gluing segment of the component enables the component to bind to another components and may carry indexing information comprising a position of the component relative to other components in the subset For the purpose of this disclosure, components may be either naturally occurring or artificially synthesised nucleic acids, such as: oligonucleotides, DNA fragments, plasmids, TNA, peptides, small metabolites, any other either organic or inorganic molecules, which are capable of representing data, or any combination of items listed herein.
set: the set comprises a plurality of (pre-prepared) components, some of which may be selected to represents data. The plurality of components in the set may include a certain combinatorial space comprising a plurality of permutations of data segments combined with a plurality of gluing segments which enable the components, to which the gluing segment is bound to, to bind to another component, and/or may carry indexing information comprising data on a position of the component relative to other components in the subset
**data segment (pl. data segments):** shall be considered as a) a part of the encoded digital data to be represented by the subset of components using the device according to the present disclosure, or b) a part of the component which represents data;
**gluing segment (pl. gluing segments):** shall be considered as a hybridisation or annealing region of the component, or an indexing part of the component that includes data on the position of the components relative to other components The gluing segment enables data segments to be indexed or placed at a certain position within a final nucleic acid molecule. The gluing segment of one component may be complementary to the gluing segment of another component in the set. For the purpose of this disclosure, the gluing segment may be any of the following: a single stranded nucleotide overhang, a sticky end, a homologous end region, or any other segment of a molecule, which is capable of carrying indexing information or assembling and binding the component to another component..
**final nucleic acid molecule (pl. final nucleic acid molecules):** the final nucleic acid molecule comprises two or more components assembled in a correct order. The final nucleic acid molecule represents data. The final nucleic acid molecule comprises the subset of components selected from the set of components and represents data.

### BRIEF DESCRIPTION OF DRAWINGS

Figure 1 schematically depicts a front view of an example embodiment of the device according to the present disclosure.
Figure 2 schematically depicts a front view of a device comprising two arrays of liquid dispensers and a processing chamber.
Figure 3A schematically depicts a top view of an example embodiment of the device comprising of three (3) arrays of liquid dispensers.
Figure 3B schematically depicts a top view of an example embodiment of the device comprising four (4) arrays of liquid dispensers.
Figure 4A schematically depicts a top view of an example embodiment of the device according to the present disclosure whereat a plurality of arrays are stacked next to each other.
Figure 4B schematically depicts a front view of an example embodiment of the device wherein a first array and a second array are positioned so that there is a slight vertical angle between the first array and the second array.
Figure 5 depicts a front view of an example embodiment of the device comprising electric plates.
Figure 6 depicts a front view of an example embodiment of the device wherein a drop collecting element is a large container.
Figure 7 schematically depicts main steps of the method.

### DETAILED DESCRIPTION

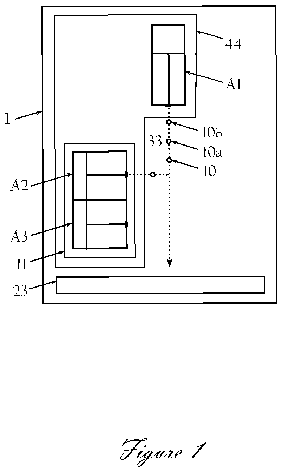

Example embodiments of devices and methods according to the present disclosure relate to a device 1 and a method 100 for recording data in nucleic acids. A detailed description of the device 1 for recording data in nucleic acids (hereinafter: the device 1) and the method 100 for recording data in nucleic acids (hereinafter: the method 100) according to the present disclosure shall be given hereinafter based on the Figures 1-7. The terminology is herein used to describe specific embodiments of the devices 1 and the methods 100, but their usage does not delimit the invention, except as outlined in the claims.

Figure 1 schematically depicts a front view of an example embodiment of the device 1 according to the present disclosure. At least one example embodiment of the device as schematically depicted in Figure 1 comprises a plurality 44 of liquid dispensers A1-A3 being configured to dispense drops, wherein at least a carrier drop 10 sequentially collides with at least drops that aggregately comprise a subset of components from a set of nucleic acid components, so that the subset of components is located in the carrier drop 10 during flight and represents data, and a drop collecting element 23 being configured to collect the carrier drop 10. Each of the drops that aggregately comprise the subset of components includes one component from the subset, suspended at a known concentration. The set of nucleic acid components (hereinafter: the set of components) comprises a plurality of either naturally occurring or artificially synthesised components, such as: oligonucleotides, DNA fragments, plasmids, TNA, peptides, small metabolites, any other either organic or inorganic molecules, which are capable of representing data, or any combination of components listed herein The set of components is held in at least some liquid dispensers in the plurality 44 of liquid dispensers in the device 1, and each component from the set may be suspended at a known concentration in a liquid held a separate container in at least one liquid dispenser from the plurality 44 of liquid dispensers. A preferred example embodiment of the device includes more than two hundred (200) liquid dispensers in the plurality 44. Components in the subset are selected from the set, so that the components in the subset represent data, and by sequentially colliding and merging the carrier drop 10 with the drops that aggregately comprise the subset of components, all of the components in the subset, which represent data, are physically located in the carrier drop 10 before the carrier drop 10 lands on the drop collecting element 23. In this way, the subset of components may be located in the carrier drop 10 and represents data before the carrier drop 10 lands on the drop collecting element 23.

In at least some example embodiments, such as depicted in Fig. 1, the device 1 comprises one liquid dispenser A1 being configured to dispense the carrier drop 10. In at least some example embodiments, the device 1 comprises more than one liquid dispenser being configure to dispense the carrier drop 10.

In at least some example embodiments of the device 1, such as depicted in Fig. 1, the carrier drop 10 has a trajectory that passes by at least two liquid dispensers 2, 3 configured to dispense drops towards the trajectory of the carrier drop 10 in a synchronised manner, so that the carrier drop 10 sequentially collides with at least all the drops that aggregately comprise components from the subset and are dispensed by the at least some of the two liquid dispensers A2, A3.

In at least some example embodiments, such as depicted in Fig. 1, the device 1 comprises the liquid dispenser A1 being configured to dispense a stream 33 of carrier drops 10 (10a,10b...). The stream 33 comprises a plurality of carrier drops 10 (10a,10b...) dispensed one after another. The stream 33 may include a first carrier drop 10, a second carrier drop 10a and at least a third carrier drop 10b. The stream 33 may be continuous or discontinuous. All of the drops are dispensed. In some example embodiments of the device 1, a stream of drops, which are not the carrier drop 10, may be dispensed. In at least some example embodiments, the device 1 comprises the plurality 44 of liquid dispensers being configured so that the first carrier drop 10 in the stream 33 collides with at least drops that aggregately comprise a first subset of components, the second carrier drop 10a in the stream 33 collides with at least drops that aggregately comprise a second subset of components, the third carrier drop 10b in the stream 33 collides with at least drops that aggregately comprise a third subset of components, and so on, until all of the carrier drops 10 (10a,10b...) in the stream 33 collide with at least some drops that aggregately comprise at least some components, which represent data, from the set, and all of the carrier drops 10 (10a, 10b...) in the stream 33 land on the drop collecting element 23. In this way, a plurality of distinct carrier drops 10 (10a, 10b...) may be produced, wherein each distinct carrier drop 10 includes one subset of components, which represent data, from the set. The first subset of components may represent some data to be recorded in nucleic acids using the device 1 and the method 100, the second subset of components may represent some other data to be recorded in nucleic acids using the device 1 and the method 100, and so on. In some example embodiments, the first subset and the second subset may represent the same data. All of the drops are dispensed.

In at least some example embodiments, the device 1 comprises at least two liquid dispensers arranged in an array, such as a linear array, a circular array, a mesh array, or any other suitable type of array comprising at least two liquid dispensers arranged in an ordered manner. In at least some example embodiments, such as depicted in Fig. 1, the device 1 comprises at least two liquid dispensers A2,A3 arranged in a first array 11, which is a linear array. In some preferred example embodiments of the device 1, the carrier drop 10 is dispensed in a direction along a plurality of nozzles in the at least two liquid dispensers A2,A3, which may be arranged in the first array 11, in a way so that the carrier drop 10 passes by the plurality of nozzles in the first array 11 in close proximity during flight. The at least two liquid dispensers A2,A3 in the first array 11 dispense drops in a timely manner towards a passing-by carrier drop 10, so that the drops that aggregately comprise components from the subset and are dispensed from the at least two liquid dispensers A1,A2 that may be arranged in the first array 11 sequentially collide and merge with the passing-by carrier drop 10 in-flight. In this way, the carrier drop 10 may collide and merge with a predefined number and combination of the drops dispensed by at least two liquid dispensers A1,A2 that may be arranged in the first array 11, whilst the carrier drop 10 is in-flight. The drops that aggregately comprise the subset of components and are dispensed from the at least two liquid dispensers, which may be arranged in the array, collide and merge sequentially with the carrier drop 10 during the carrier drop's 10 flight towards the drop collecting element 23.

In at least some example embodiments, each of the liquid dispensers in the plurality 44 in the device 1 comprises at least a container for holding liquids to dispense, a drop generating element positioned and configured to cause at least one drop to dispense, a nozzle through which the at least one drop is dispensed from, a capillary which connects the container to the nozzle so that the liquid runs from the container through the capillary and out through the nozzle for the at least one drop to be dispensed, and synchronisation means, which enable the each of the liquid dispensers to operate in a synchronised manner with other liquid dispensers in the plurality 44 of liquid dispensers in the device 1. In at least some example embodiments, the drop generating element is a piezo element. In such example embodiments, the synchronisation means are configured to apply voltage to the piezo element at a pre-determined moment in time, so that the piezo element deforms and pushes the liquid out through the nozzle, and in this way, the at least one drop is dispensed at a correct time and in the synchronised manner with the other liquid dispensers in the plurality 44. The voltage applied to the piezo element determines the deformation of the piezo element and consequently a size of the at least one drop and a speed at which the at least one drop is dispensed. In at least some example embodiments, the drop generating element is a thermal element configured to change the temperature of the liquid used for dispensing drops. In such example embodiments, the synchronisation means are configured to control the thermal element so that the thermal element increases or decreases the temperature of the liquid used for dispensing the at least one drop in a pre-determined moment in time. When the synchronisation means control the thermal element so that the thermal element causes the temperature of the liquid to increase, said liquid expands in volume and thus ejects out through the nozzle, and in this way, at least one drop is dispensed. The change in temperature and consequently expansion of the volume of the liquid determines the size of the at least one drop and the speed at which the at least one drop is dispensed. In at least some example embodiments, the drop generating element is any other electro-mechanical element capable of producing a mechanical movement which pushes the liquid out through the nozzle, or any other thermal element configured to change the temperature of the liquid so that the liquid expands and is thus ejected through the nozzle. In at least some example embodiments, a single container is configured to supply liquids to two or more nozzles in the plurality 44 of liquid dispensers in the device 1. In at least some example embodiments, two or more containers are configured to supply liquids to a single nozzle in the plurality 44 of liquid dispensers in the device 1. All liquid dispensers in the plurality 44 of liquid dispensers operate in the synchronised manner.

Each of the components in the set is suspended at a known concentration in a liquid, which is held in at least one separate container that supplies the liquid to at least one liquid dispenser in the plurality 44 of liquid dispensers in the device 1. All of the components from the set are suspended in liquids held in separate containers in at least some of liquid dispensers in the plurality 44 of liquid dispensers. The components may be suspended in water, specific buffer with appropriate pH, other aqueous solution, or any other liquid capable of holding components intact. Components in the set may be pre-prepared in a way so that gluing segments are added to the data segments of components. Each component from the set may therefore comprise a data segment that represents the data, and at least one gluing segment, which may enable the component to assemble and bind to other components from the subset in a correct order, or may include indexing information including the position of the component, to which the gluing segment is bound to, relative to other components in the subset. For the purpose of this disclosure, the term 'data segment' shall be used herein to refer to a segment the component that represents data, and the term 'gluing segment' shall be used herein to refer to an indexing part or a hybridisation part of the components, as the gluing segment may comprise indexing information on the position of the component relative to other components ,or may enable the the component to assemble and bind to other components. To record data in nucleic acids using the device 1 and the method 100, components from the subset may be indexed in a row of successive components (e.g. one after another) in the correct order in the subset, by using the gluing segments, regardless of whether or not the components are assembled and bound together in the correct order to form final nucleic acid molecules, and the gluing segments enable indexing the components in the correct order in the subset, even in the example embodiments wherein the components are not assembled and bound together to form the final nucleic acid molecules. The gluing segments may in some example embodiments be complementary single stranded overhangs.

The subset may comprise, for example, eight (8) components, so that eight (8) possible final positions for components are available in the subset. The set may be pre-prepared from, for example, ten (10) distinct data segments, which are capable of being positioned in any of the eight (8) possible final positions available in the subset, which comprises eight (8) components. The set, in this given example, may be pre-prepared in a way so that the set comprises eighty (80) components, wherein each of the ten (10) distinct data segments was assembled and bound together with each of the eight (8) distinct gluing segments that allow for the eight (8) possible final positions in the subset, and in this way, eighty (80) components were pre-prepared to be included in the set. Each of the eighty (80) components is suspended in a liquid at a known concentration and is held in an individual container which supplies the nozzle in at least one liquid dispenser in the plurality 44 of liquid dispensers in the device 1. In this example, the device 1 comprises at least eighty-one (81) liquid dispensers, wherein eighty (80) liquid dispensers are holding liquids each comprising one out of the eighty (80) components from the set, and one liquid dispenser is configured to dispense the carrier drop 10. In the given example, the plurality 44 of liquid dispensers is configured so that the carrier drop 10 is dispensed and sequentially collides and merges with at least eight (8) drops each comprising one component out of the eight (8) components from the subset, which represents data, before the carrier drop 10 lands on the drop collecting element 23, so that all of the eight (8) components from the subset are located in the carrier drop 10 before the carrier drop 10 lands on the drop collecting element 23. A correct sub-plurality of eight (8) liquid dispensers are configured to dispense the eight (8) drops that aggregately comprise the subset of eight (8) components and are dispensed at correct pre-determined moments in time, so that the eight (8) drops collide and merge with the carrier drop 10 during flight. All of the eight (8) drops that aggregately comprise the subset of the eight (8) components, sequentially collide and merge with the carrier drop 10 in the synchronised manner, so that a sequence of successive collisions occurs over time, wherein each collision in the sequence of successive collisions causes one component (from the subset comprising the eight (8) components) to be collected by the carrier drop 10. The sequence of successive collisions between the carrier drop 10 and the eight (8) drops that aggregately comprise the subset of eight (8) components includes a first collision between the carrier drop 10 and a first drop that includes a first component from the subset, a second collision between the carrier drop 10 and a second drop that includes a second component in the subset, a third collision between the carrier drop 10 and a third drop that includes a third component in the subset, and so on, until all of the eight (8) drops that aggregately comprise the subset of the eight (8) components, collide and merge with the carrier drop 10. In this way, the eight (8) components in the subset that represents data, are positioned together and located in one location, in the carrier drop 10, before the carrier drop 10 lands on the drop collecting element 23. It is understood that the subset of components that represents data may include any suitable number or combination of components from the set, and the set may include any number of components, depending on the example embodiment of the device 1 and method 100. Any number of carrier drops 10 (10a, 10b...) may be dispensed in the stream 33, and the sequence of successive collisions may be performed for each of the carrier drops 10 (10a, 10b...) in the stream 33, and it is understood. that a different subset of components may be collected by each of the carrier drops 10 (10a, 10b...) in the stream 33.

In at least some example embodiments, the device 1 may include each of the liquid dispensers in the plurality 44 of liquid dispensers being configured to dispense up to a hundred thousand (100000) drops per second. In at least some example embodiments, the device 1 may include each of the liquid dispensers in the plurality 44 of liquid dispensers being configured to dispense drops with a volume in a range between one (1) picoliter to a hundred (100) nanoliters. In at least some example embodiments, the device 1 may include fifty or more, or five hundred or more, or ten thousands or more liquid dispensers in the plurality 44.

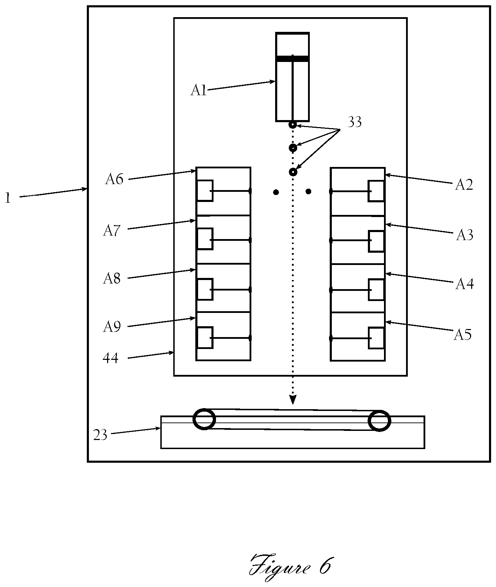

The drop collecting element 23 may be configured to collect the carrier drop 10 immediately after the carrier drop 10 collides and merges with the drops that aggregately comprise the subset of components. At the moment of landing on the drop collecting element 23, the carrier drop 10 comprises at least all components from the subset that represents data. In at least some example embodiments, the device 1 may include the drop collecting element 23 made of a non-bendable material (such as glass, plastics and other non-bendable materials). In at least some example embodiments, the device 1 may include the drop collecting element 23 made out of a bendable material. In at least some example embodiments, the device 1 may include the drop collecting element 23, which is a surface, which may be flat. In at least some example embodiments, the device 1 may include the drop collecting element 23 that is detachable relative to the device 1. In at least some example embodiments, the device 1 may include the drop collecting element 23 that is non-detachable relative to the device 1. In at least some example embodiments, the device 1 may include the drop collecting element 23, which is a container filled with a liquid (such as schematically depicted in Figure 6) in which the carrier drops 10 (10a, 10b...) land. In at least some example embodiments, the device 1 may include a submersion element installed and configured to submerse the carrier drops 10 (10a, 10b...) in the liquid held in the container, which is the drop collecting element 23 in this example, immediately after the carrier drops 10 (10a, 10b...) land in said liquid held in the container. In at least some example embodiments, the device 1 may include the drop collecting element 23 with a plurality of wells, and the carrier drops 10 (10a, 10b...) land in the plurality of wells in the drop collecting element 23.

In at least some example embodiments, the device 1 may include the plurality 44 of liquid dispensers being configured to dispense drops of a first liquid, and the drop collecting element 23 being filled or covered with a second liquid, and the first liquid and the second liquid are immiscible. The first liquid and the second liquid are immiscible and do not mix with each other, and the carrier drops 10 (10a, 10b...) remain intact, do not merge amongst each other, and are protected from evaporation immediately after landing in the second liquid, which located in or on the drop collecting element 23. The second liquid may surround or form a coating surrounding the carrier drops 10 (10a, 10b...) immediately after the carrier drops 10 (10a, 10b...) land in the second liquid. In at least some example embodiments, the device 1 comprises the plurality 44 of liquid dispensers being configured to dispense drops of an aqueous solution, and the drop collecting element 23 may be filled or covered with a hydrophobic liquid, which may be oil-based and may have a low volatility, so that the carrier drops 10 (10a,10b...) made of the aqueous solution may remain intact, may not merge amongst each other, and may be protected from evaporation immediately after landing in the hydrophobic liquid located in or on the drop collecting element 23. The hydrophobic liquid may surround or form a coating surrounding the aqueous solution of the carrier drops 10 (10a, 10b...), and in this way, such application of the hydrophobic liquid to the drop collecting element 23 may enable that the carrier drops 10 (10a, 10b...) do not mix amongst each other, remain intact, and do not evaporate after landing. It is understood that hydrophobic liquids may also be used to cover the surface of the drop collecting element 23 in the example embodiments wherein the drop collecting element 23 comprises the surface.

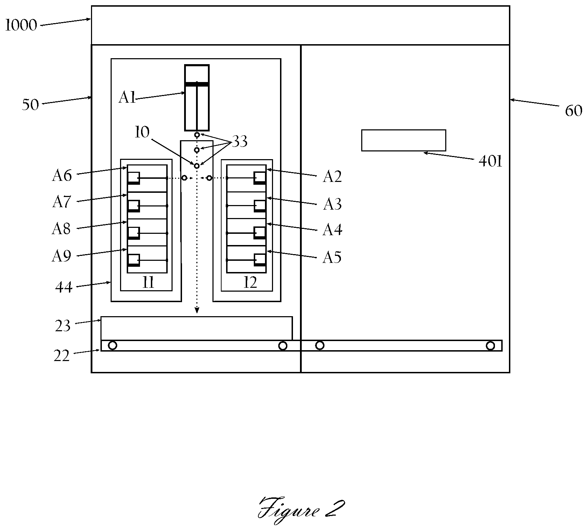

In at least some example embodiments of the device 1, such as depicted in Figure 2, at least some liquid dispensers A2-A9 in the plurality 44 of liquid dispensers A1-A9 are arranged in two arrays, the first array 11 and a second array 12. In the example embodiment of the device 1, such as depicted in Figure 2, the first array 11 and the second array 12 dispense drops towards a single stream 33 of carrier drops 10 (10a, 10b...). In the example embodiment of the device 1, such as depicted in Figure 2, the first array 11 and the second array 12 dispense drops in a horizontal direction towards a vertical trajectory of the carrier drop 10, which is dispensed by the liquid dispenser A1, so that drops dispensed from the first array 11 and the second array 12 collide with the carrier drop 10 at a ninety (90) degree angle. It is understood that other angles between the trajectories of the drops dispensed by the at least some liquid dispensers A2-A9 and the trajectory of the carrier drop 10 may be utilised in at least some example embodiments of the device 1 and method 100 according to the present disclosure.

In at least some example embodiments of the device 1, at least the first array 11 may configured to dispense drops towards at least a first stream 33 of carrier drops 10 (10a,10b...), and at least the second array 12 is configured to dispense drops towards at least a second stream of carrier drops 10 (10a, 10b...). It is understood that any suitable number of arrays, such as the at least first array 11 and the second array 12, or any number of streams 33 of carrier drops 10 (10a,10b...) may be used with the device 1.

In at least some example embodiments of the device 1, such as depicted in Figure 2, the first array 11 and the second array 12 are facing each other so that the angle between them is a hundred and eighty (180) degrees observing from above. In at least some example embodiments of the device 1 and the method 100 according to the present disclosure, a slightly smaller or larger angle than the hundred and eighty (180) degrees may be utilised for the purpose of avoiding a collision between the carrier drop 10 and any possible satellite droplets which may be produced as a result of collisions between the carrier drops 10 (10a, 10b...) with other drops dispensed from at least the first array 11 or as a result of inaccurate operation of any of the liquid dispensers in the device 1.

In at least some example embodiments, such as as depicted in Figure 2, the device 1 comprises a mixing chamber 50 wherein the plurality of liquid dispensers A1-A9 and at least the drop collecting element 23 are positioned. The mixing chamber 50 is a partially or a fully closed compartment of the device 1. The mixing chamber 50 is the fully closed compartment of the device 1 during the time in which the drops are in-flight, so that the drops evaporate less that in a fully open environment. The mixing chamber 50 is a partially closed compartment during the time in which the drop collecting element 23 is added or removed from the mixing chamber 50. In at least some example embodiments, the device 1 comprises a pressure regulator positioned in the mixing chamber 50 and configured to control the pressure in the mixing chamber 50. In at least some example embodiments of the device 1, the pressure regulator installed in the mixing chamber 50 is configured to produce a higher pressure or a lower pressure (relative to an atmospheric pressure) in the mixing chamber 50, by adding or removing gases to or from the mixing chamber 50 so that the higher pressure or the lower pressure in the mixing chamber 50 may be achieved this way. In at least some example embodiments, the device 1 comprises a cleaning element installed in an interior of the mixing chamber 50 and is configured to clean the interior of the mixing chamber 50 during a cleaning protocol. In at least some example embodiments, the cleaning element may be any appropriate element which is capable of cleaning an interior of the mixing chamber 50, such as: a light source, an UV light source, a source of liquids, a source of gases, and any other source of materials or radiation (such as electromagnetic radiation or acoustic radiation or a combination hereof) which may be used to clean the interior of the mixing chamber 50.

In at least some example embodiments, such as depicted in Figure 2, the device 1 comprises a mechanisation 22. The mechanisation 22 may include any appropriate number and combination of electro-mechanical, mechanical, or software parts, that enable a mechanical movement in at least one direction. Parts that are included in the mechanisation 22 may be parts, such as: electrical motors (such as stepper motors or other motors), belts (such as GT2 belt or other belts), couplers, bearings, gears, shafts, rods, linear actuators, pneumatic actuators, guides (such as linear motion guides or other guides), rods, rails (such as linear rails or other rails), conveyor belts, control signal generators (such as PWM signal generators or other control signal generators), application specific software (such as firmware), microcontrollers, any combination of items listed herein, or any other electro-mechanical, mechanical, or software parts, which are capable of moving the drop collecting element 23 (or in some example embodiments the plurality 44 of liquid dispensers) in at least one direction in the device 1.

In at least some example embodiments of the device 1, such as depicted in Figure 2, the device 1 comprises the mechanisation 22 being configured to move the drop collecting element 23 in at least one direction relative to the plurality 44 of liquid dispensers A1-A9. In at least some example embodiments, the device 1 comprises the mechanisation 22 being configured to move the drop collecting element 23 in three (3) axis relative to the plurality 44 of liquid dispensers. In at least some other example embodiments, the device 1 comprises the mechanisation 22 being configured to move the plurality 44 of liquid dispensers in at least one direction relative to the drop collecting element 23. Moving the drop collecting element 23, by using the mechanisation 22 23, relative to the plurality 44 of liquid dispensers A1-A9 (Fig. 2), may be performed during the collection of the plurality of distinct carrier drops 10 (10a, 10b...) onto the drop collecting element 23 in a way wherein each of the distinct carrier drops 10 (10a, 10b...) in the plurality lands on a single and unique pre-determined landing coordinate on the drop collecting element 23.

In at least some example embodiments of the device 1, the carrier drop 10 includes binding reagents, which enable an assembly and binding of the components located in the carrier drop 10. One container, which is supplying liquids to the liquid dispenser A1, may include the binding reagents. In at least some example embodiments of the device 1, the binding reagents are provided by a drop that collides and merges with the carrier drop 10 during flight.

In one example, after all of the carrier drop 10 in the stream 33 land on the drop collecting element 23, the drop collecting element 23 may be removed from the device 1. In such example embodiments, a final product of the device 1 and the method 100 is the plurality of distinct carrier drops 10 (10a, 10b...) located on the drop collecting element 23, wherein each of the distinct carrier drops 10 (10a, 10b...) includes at least some components, which represent data, from the set, and may also include the binding reagents.

In at least some example embodiments, such as depicted in Fig 2., the device 1 comprises a processing chamber 60. The mechanisation 22 may be configured to move the drop collecting element 23 from the mixing chamber 50 to the processing chamber 60. In some examples, a user of the device 1 may move the drop collecting element 23 to the processing chamber 60, after all carrier drops 10 (10a, 10b...) in the stream 33 land on the drop collecting element 23. The processing chamber 60 is a fully or a partially closed compartment of the device 1. The processing chamber 60 may be located either next to, below or above the plurality 44 of liquid dispensers, or apart in a a separate location. The processing chamber 60 may be the fully closed compartment during a time in which a biochemical reaction occurs in each carrier drop 10 positioned in the processing chamber 60. The processing chamber 60 may be the partially closed compartment during the time in which the drop collecting element 23 is added or removed to or from the mixing chamber 50. In the processing chamber 60, at least one temperature regulator 401 is installed and configured to change a temperature in the processing chamber 60 over time so that a sequence of temperatures is achieved over time in the processing chamber 60, and the sequence of temperatures causes the biochemical reaction to occur between the components and binding reagents located in each of the carrier drops positioned in the processing chamber 60. The temperature regulator 401 is therefore used to heat or cool the controlled environment in the processing chamber 60 over time and according to the sequence of temperatures that causes the biochemical reaction. The sequence of temperatures may include at least a first temperature held for a pre-determined amount of time in the processing chamber 60, a second temperature held for a pre-determined amount of time in the processing chamber 60, and so on, until all temperatures are achieved in the sequence of temperatures and the biochemical reaction occurs successfully in each of the carrier drops 10 (10a,10b...) positioned in the processing chamber 60. The biochemical reaction causes the components located in each of the carrier drops 10 (10a, 10b...)to assemble and bind together to form the final nucleic acid molecules, which represent data. The final nucleic acid molecules comprise two or more components assembled and bound together in the correct order. The biochemical reaction may be any appropriate biochemical reaction, which causes the components to assemble and bind together, such as: Polymerase Chain Reaction (hereinafter: PCR), Sticky End Ligation, Cell Free Cloning, BioBricks Assembly, Gibson Assembly, HiFi Assembly, *in vivo* cloning methods, or any other appropriate biochemical reaction which may be used to assemble and bind the components together. The biochemical reaction may include multiplication of the final nucleic acid molecules.

In at least some example embodiments, wherein the biochemical reaction used with the device 1 and the method 100 disclosed herein is the PCR, the binding reagents may include at least a polymerase enzyme, dNTPs, appropriate buffer and potentially other co-factors and supplements to optimize the PCR and are included in the carrier drops 10 (10a, 10b...) at the time of dispensing or may be provided by the drop that collides with the carrier drop. In at least some example embodiments, wherein the biochemical reaction used with the device 1 and the method 100 disclosed herein is the PCR, the binding reagents may include a standard PCR Master Mix liquid. The binding reagents are included in the carrier drop 10 or provided by the drop that collides and merges with the carrier drop 10 during flight, as discussed previously. In example embodiments wherein the PCR is used, the temperature regulator 401 in the processing chamber 60 provides the sequence of temperatures over time, so that the PCR occurs and the final nucleic acid molecules are synthesised from the components in the presence of the binding reagents (which may include the PCR Master Mix fluid in this example) located in the each of the carrier drops 10 (10a, 10b...) positioned in the processing chamber 60. In example embodiments wherein the PCR is used with the device 1, the components or the final nucleic acid molecules may also be multiplied.

In at least some example embodiments wherein the biochemical reaction used with the device 1 and the method 100 disclosed herein is the Cell Free Cloning, the binding reagents may include at least all necessary *in vitro* purified enzymes needed to reconstitute bacterial (*E. Coli*) replication mechanism and may be included in the carrier drop 10 or provided by the drop that collides and merges with the carrier drop 10 during flight. By using the Cell Free Cloning with the device 1 and the method 100, the final nucleic acid molecules may be plasmids. In at least some example embodiments, at least one liquid dispenser is configured to dispense drops that comprise the binding reagents, which include liquids or molecules that aid the *in vitro* cell-free DNA fragment assembly process, wherein the binding reagents are included in the carrier drop 10 provided by the drop that collides and merges with the carrier drop 10 during flight, as discussed previously. In at least some example embodiments, wherein the biochemical reaction used with the device 1 and the method 100 disclosed herein is the Sticky End Ligation, at least one liquid dispenser is configured to dispense drops that comprise the binding reagents, which include a buffer comprising a ligase enzyme and other substrates to aid the Sticky End Ligation occurring between the components. The binding reagents are included in the carrier drop 10 or provided by the drop that collides and merges with the carrier drop 10 during flight. In the example embodiments wherein the Sticky End Ligation is used with the device 1, the components may bind in the presence of the binding reagents at a room temperature, if a correct concentration of the ligase enzyme, components, and other substrates to aid the biochemical reaction are all located in the carrier drop 10. In such example embodiments, the biochemical reaction may occur in another location, which is not the processing chamber 60, and the drop collecting element 23 may be removed from the device 1 and placed in the another location after all of the carrier drops 10 (10a, 10b...) that include the components and the binding reagents, land on the drop collecting element 23. In example embodiments wherein the biochemical reaction, which is used with the device 1 and the method 100 hereof, is any other appropriate biochemical reaction which may be used to assemble and bind the components from the subset together to form the final nucleic acids, any suitable binding reagents may be included in the carrier drop 10 (at the time of dispensing) or provided by the drop that collides with the carrier drop 10 before landing, and the any suitable binding reagents may include substances to aid the assembly and binding of components during the any other appropriate biochemical reaction.

In at least some example embodiments, the device 1 may include at least one light regulator mounted in the processing chamber 60 and configured to regulate the amount of light in the processing chamber 60 over time. The at least one light regulator in the processing chamber 60 may comprise a source of visual light or a source of UV light that may be used during the cleaning protocol of the interior of the processing chamber 60. In at least some example embodiments, the device 1 comprises a humidity regulator mounted in the processing chamber 60 and configured to regulate humidity in the processing chamber 60 over time.

In at least some example embodiments, the device 1 may include a user interface being configured to provide user data. The user interface may include any number of electro-mechanical, mechanical or software based user interface parts, which are available now or which shall become available in the future, such as: a display, a touch screen, a computer keyboard, a touch sensor of any kind, knobs, buttons, a camera, a computer, a body sensor, devices for visually or otherwise impaired, a computer mouse, or other. The user interface allows the user of the device 1 to provide the user data, which include but are not limited to: a) data to be recorded in nucleic acids by using the device 1 and the method 100, b) method 100 settings, b) device 1 settings and other relevant user data. Data to be recorded in nucleic acids by using the device 1 and the method 100 hereof, may include digital data in any digital format available, including but not limited to compressed files. The digital data may in some examples include metadata information related to the digital data itself. Those skilled in the arts shall recognise that examples of metadata are: a date of creation of the digital data, format type of the digital data, compression parameters of the digital data, authors, copyright notices, encoding parameters and settings, location data and other similar metadata attached to the digital data to be recorded in nucleic acids using the device 1. The user of the device 1 may be a human, a robot, a software, a remotely-controlled software, or any other entity capable of inputting data to the user interface or any combination of the herein listed options.

The method settings provided by the user may include settings related to the method 100, such as:
a) the biochemical reaction used with the device 1 and the method 100, wherein the biochemical reaction may be: Restriction / Ligation (Sticky End Ligation or Blunt End Ligation), Polymerase Chain Reaction (PCR), Cell Free Cloning Systems, BioBricks assembly, Golden Gate assembly, Gibson assembly, homologues recombination, HiFi assembly, in vivo cloning methods, any other biochemical reaction, which may assemble and bind the components together;
b) the method 100 settings related to the biochemical reaction: choosing the type of liquids to be used for dispensing drops, defining a concentration of components in liquids, defining the binding reagents, defining liquid parameters (such as viscosity, storage temperature, pH, other), defining the sequence of temperatures to be provided by the temperature regulator 401 in the processing chamber 60, temperature and duration of process cycles in the processing chamber 60 during the biochemical reaction, temperature in the mixing chamber 50 during the time in which the drops and in-flight, and any other settings related to the method 100;
c) defining an encoding algorithm and encoding settings to be used for encoding and mapping of the digital data : the n value (discussed later in this document), number of bits used in digital data encoding, the length of the data segments available in the set, the length of the gluing segments available in the set, metadata, look up tables, or other possible parameters of the encoding method 100. It is understood to those skilled in the arts that information related to the encoding and mapping may include data correction mechanisms, look up tables, and other relevant encoding and mapping setting.

The device settings provided by the user may include but are not limited to:
a) settings of the liquid dispensers, such as: a number of liquid dispensers used in the device 1, the sizes of the at least one drop, sizes of all dispensed drops, the speed of the at least one drop, speeds of all the dispensed drops, size of the containers that supply liquids, and other similar settings.
b) settings of the drop collecting element 23 such as: the size and position of the drop collecting element 23, location and movement range of the drop collecting element 23, possible size and position of the wells on the drop collecting element 23, the distance between and locations of said wells, and other settings of the drop collecting element 23
c) the mechanisation 22 settings, such as: the number of axis, movement range, acceleration parameters etc.,
d) processing chamber 60 settings, such as: the sequence of temperatures provided by the temperature regulator 401, settings related to binding reagents and concentration of components, a buffer pH or addition of cofactors (ATP) to ligation mixture in the case wherein Sticky End Ligation is used, the temperature/pH in the case wherein the Cell Free Cloning system is used., other setting in the processing chamber 60 such as light, humidity, pressure, pH, addition of cofactors, cleaning protocols, or any other relevant setting related to the processing chamber 60.

In at least some example embodiments, such as depicted in Figure 1, the device 1 comprises an inlet. The inlet may simply be a door which is configured to open or close in order for the user of the device 1 to add or remove at least the drop collecting element 23 to or from the device 1. In at least some example embodiments, the inlet is a separate compartment of the device 1, which may be located next to, above or below an operational space of the mixing chamber 50. In at least some example embodiments, the inlet is a compartment of the device 1 whereat the plurality of drop collecting elements 23 may be stacked before the device 1 starts recording the data.

In at least some example embodiments, such as depicted in Figure 2, the device 1 comprises an outlet. The outlet may simply be a door which is configured to open or close in order for the user of the device 1 to add or remove at least the drop collecting element 23 to or from the device 1. In at least some example embodiments, the outlet is a separate compartment of the device 1, which may be located next to, above or below the operational of the mixing chamber 50. In some example embodiments, the outlet is a compartment of the device 1 whereat the plurality of drop collecting elements 23 may be stacked until the device 1 completes all operations and before the user removes the plurality of drop collecting elements 23 from the device 1. In at least some example embodiments, the inlet and the outlet are the same door or compartment of the device 1.

In at least some example embodiments, such as depicted in Figure 2, the device 1 comprises a control unit 1000 configured to control parts of the device 1, such as the plurality 44 of liquid dispenses, the mechanisation 22, or other herein disclosed parts of the device 1. The control unit 1000 may include parts such as: at least one computer which may comprise all standard computer parts such as, but not limited to: at least one data processor, at least one memory storage, at least one data bus, at least one data input and output connecting means, all software parts (such as application-specific software including firmware ) and other. In at least some example embodiments, the control unit 1000 may include parts such as: a power storage, a power supply, at least one micro-controller, at least one AD/DA converter, at least one power signal converter, at least one power or control signal amplifier at least one cable, at least one connector for the at least one cable, and may include other electro-mechanical or mechanical parts which are needed to: a) connect the control unit 1000 physically to other parts of the device 1 (such as the plurality 44 of liquid dispensers , the mechanisation 22 or any other herein disclosed parts of the device 1), and b) connect the control unit 1000 to other device 1 parts (such as any of the device 1 parts depicted in Figure 1 or 2) with data connecting means. The control unit 1000 may be configured to generate each and every control signal that is used for controlling each and every part of the device 1 (such as the plurality 44 of liquid dispensers or other parts of the device 1).

The control unit 1000 may send each of the generated control signals to a dedicated part of the device 1 (such as any one liquid dispenser in the plurality 44) via the data output connecting means, and when each of the generated control signals is received by the dedicated part of the device 1, the dedicated part of the device 1 functions according to the control signal sent from the control unit 1000 to the dedicated part of the device 1.

The control unit 1000 may be configured to control all of the drop generating elements in the plurality 44 of liquid dispensers, so that all drop are dispensed at correct moments in time and all collisions (between the carrier drop 10 and other drops) occur, which result in at least the subset of components being located in the carrier drop 10, and all liquid dispensers in the plurality 44 of liquid dispensers operate in the synchronised manner. In at least some example embodiments, the control unit 1000 may include at least one peripheral control unit configured to control the operation of at least some parts in the device 1. In some example embodiments, the peripheral control unit is a cartridge control system used for controlling parts in the plurality 44 of liquid dispensers.. The cartridge control system is a part of the control unit 1000 and is configured to control the plurality 44 of liquid dispensers in the synchronised manner. Any of the peripheral control units, such as the cartridge control system, may contain similar parts as contained in the control unit 1000, including but not limited to micro-controllers, local memory storage, processing units, or other appropriate parts, which may be used for controlling any of the operational parts of the device 1. In at least some example embodiments, the device 1 comprises the control unit 1000 being configured to control the plurality 44 of liquid dispensers, the drop collecting element 23, and/or the mechanisation 22, and/or the mixing chamber 50, and/or the temperature regulator 401 in the processing chamber 60, or any other operational parts of the device 1.

In at least some example embodiments, the device 1 may include the control unit 1000 being configured to encode and map the data to be recorded in nucleic acids using the device 1 and the method 100 according to the present disclosure. The encoding and mapping, by using the control unit 1000, shall be discussed hereinafter in more detail. The data to be recorded in nucleic acids may be either in a form of digital data or analogue data. In the case wherein the data is analogue data, the analogue data is firstly converted to the digital data. The digital data is usually in the form of a string of binary symbols (0 and 1). During the encoding, the control unit 1000 may be configured to transcode the string of binary symbols (of the digital data to be recorded in nucleic acids) to a string of symbols having *n* possible values, wherein the transcoding depends on the example embodiment of the device 1 and the method 100 which is used. In at least some examples of the encoding, the *n* value is four (4) so that each out of the four (4) values may correspond to each out of four (4) naturally occurring nucleic acid bases, which may be used to represent data in each of the components in the set. In at least some examples of the encoding, the *n* value is any possible value, which is suitable to represent digital data in the components from the set. It is understood that during the encoding, an encoding algorithm may be used, wherein the encoding algorithm includes all mathematical functions and operations performed in order to convert (or transcode) the string of binary symbols to the string of symbols having *n* possible values, based on parameters of the plurality of data segments available in the set and parameters of the data, so that during the encoding, the encoding algorithm abides by: a length of the data segments available in the set, a number of the data segments available in the set, *n* number of different symbols which are used to represent data, number of bits and bytes used in the representation of digital data, and so on. In this way, during the encoding, the digital data is converted (or in a more correct word: transcoded) to the string of symbols having *n* possible values based on the parameters of the data segments available in the set and the parameters of the digital data, by using the encoding algorithm.

By using the control unit 1000, the string of distinct symbols haying *n* possible values, which was generated during the encoding, may be mapped onto a plurality of successive data segments available in the set, so that each and every subsegment of the string of symbols haying n possible values is mapped to one data segment available in the set. In this way, only a subset of data segments available in the set are chosen to represent the digital data, which was encoded, so that data segments in the plurality of successive data segments, which are mapped to represent digital data, are known. After the string of symbols having n possible values is mapped onto the plurality of successive data segments available in the set, the subset of components may be selected. Components from the subset, which is selected to represent data, aggregately comprise the data segments from the plurality of successive data segments, which represents data, and all correct gluing segments, which enable said data segments to be positioned in the correct order or indexed correctly. The mapping of the string of symbols having *n* possible values onto to the plurality of successive data segments, which are available in the set, may be based on the at least one look-up table. The look-up table may include information on the length of the data segments available in the set, the number of data segments available in the set, indexing information (including the information on the position of each component relative to any other component in the subset), encoding information, encoding algorithm, and other relevant information. It is understood to those skilled in the art that the information on the encoding and the mapping steps as well as in the look up table, may also be recorded and represented in the subset, so that the retrieval and reading back of the data, which is represented by the subset of components, may be performed in an efficient way. In some example embodiments, The device 1 comprises the control unit 1000 being configured to select the subset of components from the set, so that the subset represents data. It is understood that a plurality of subsets may be selected to represent the data.

In at least some example embodiments, the device 1 may include at least one pressure sensor configured to measure the pressure in any of the containers, or any of the capillaries, any of the nozzles, in the mixing chamber 50, in the processing chamber 60, or elsewhere in the device 1. The control unit 1000 may be configured to send or receive data from the at least one pressure sensor in real-time. In at least some example embodiments, the device 1 comprises at least a second sensor, such as: a liquid flow sensor configured to measure the flow of liquids, a temperature sensor configured to measure the temperature, a weight sensor configured to measure a weight, an optical sensor configured to gather optical measurements, an occlusion sensor, a camera configured to gather visual data, or any other sensor which may be used for measuring and gathering data at any location in the device 1. The at least second sensor may be controlled by the control unit 1000 and may be configured to send or receive data from the control unit 1000. In at least some example embodiments, the device 1 may comprise valves, such as at least one pressure valve, at least one liquid valve, micro-switches or liquid gates, which may be configured to control the flow of liquids within the plurality 44 of liquid dispensers A1-A9. In at least some example embodiments, the device 1 comprises at least one other mechanical or electro-mechanical part, such as: various liquid valves (outlet, inlet, manual, automatic, guided, etc.), T shaped ports, flanges etc., logical gates, capillary labyrinths, gates for controlling of such capillary labyrinths and other useful fluid manipulation parts. In at least some example embodiments, the device 1 comprises a second mechanisation configured to move at least one liquid dispenser in the plurality 44 of liquid dispenser to a cleaning position during the cleaning protocol, and the cleaning element may be configured to clean the at least one liquid dispenser located at the cleaning position. In at least some example embodiments, the second mechanisation is configured to move at least one liquid dispenser in the plurality 44 of liquid dispensers to a calibrated position during a calibration protocol. In an example wherein the second mechanisation is mounted in the the at least one liquid dispenser and the calibration protocol is executed, the second mechanisation may be configured to move the at least one liquid dispenser to the calibrated position wherein the nozzle of the at least one liquid dispenser is aligned and directed towards the stream 33 of carrier drops 10 (10a, 10b...). It is understood that other options of mounting the second mechanisation to individual parts of the device 1 are possible in at least some example embodiments of the device 1. In at least some example embodiments of the device 1, at least some of the liquid containers may hold a cleaning liquid used for the purpose of cleaning of the liquid dispensers during the cleaning protocol. It is understood that the container supplying one of the liquid dispensers in the plurality 44 may hold a liquid which is different than the liquid held in any of the other containers supplying liquids to other liquid dispensers in the plurality 44. Understandably, the container in the liquid dispenser A1 may hold the same liquid as the liquid stored in any of the other containers supplying liquids to any of the other liquid dispensers in the plurality 44 of liquid dispensers Such an example might be beneficial wherein the containers temporarily hold, for example, cleaning liquid.

In at least some example embodiments, the device 1 may include two or more containers being configured to supply liquids to a single nozzle in the plurality 44 of liquid dispensers. In such a case, at least one micro-fluid switch may be installed and configured to control the flow of liquids from the two or more containers through the single nozzle. The switch may be a pressure based capillary connection, or a micro-mechanical valve, a thermal based liquid switch, piezo based or other. The switch may enable the liquid from a first container to flow through and out the single nozzle, or the liquid from a second container to flow throughout the nozzle, or liquids from the first container and the second container to flow throughout to the nozzle, or neither. The switch creates a logical gate for liquids and may be controlled by the control unit 1000, and in some examples, a plurality of switches connected to different liquid dispensers in the plurality 44 of liquid dispenser in the device 1 may all be simultaneously, yet individually, controlled by the control unit 1000.

In at least some example embodiments, the device 1 may include a single container being configured to supply liquids to two or more nozzles in the plurality 44 of liquid dispensers . In at least some example embodiments, one container may be connected with all of the nozzles in at least some liquid dispensers arranged in the array, such as the first array 11, at the same time. In at least some example embodiments, a single container may be configured to supply liquids to at least one nozzle in the first array 11 and at least one nozzle in the second array 12. In at least one preferred example embodiment, the device 1 comprises a single container connected to one nozzle in each of the two or more arrays 11,12 in the device 1. In at least some example embodiments, the container in at least one liquid dispenser in the plurality 44 of liquid dispenser may be detachable.

In the example embodiment of the device 1, such as depicted in Figure 2, the first array 11 and the second array 12 are positioned at 180 degrees observing from above and are facing each other. In more detail, the first array 11 and the second array 12 are arranged so that nozzles from the liquid dispenser A2 and the liquid dispenser A6, and nozzles from the liquid dispenser A3 and the liquid dispenser A7, and nozzles from the liquid dispensers A4 and the liquid dispenser A8, and nozzles from the liquid dispenser A5 and liquid dispenser A9 are facing each other. The pairs of nozzles are positioned at the same height and are facing each other. One container may be connected to one pair of nozzles, which are positioned at the same height, such as the nozzles from the liquid dispenser A2 and the liquid dispenser A6.

In at least some example embodiments of the device 1 and the method 100 according to the present disclosure, the first array 11 and the second array 12 may be positioned at a 90-degree angle, 120-degree angle, 45-degree angle or any other suitable angle. In at least some example embodiments of the device 1 and the method 100 according to the present disclosure, the first array 11 may be configured to dispense drops towards a first stream 33 of carrier drops 10, and the second array 12 may be configured to dispense drops towards a second stream of carrier drops. In such example embodiments, the first array 11 and the second array 12 may be positioned in parallel to each other. The various example embodiments allow for customisation of the angle between the first array 11 and the second array 12.

In at least some example embodiment, containers supplying pair of nozzles that are at the same height in at least the first array 11 and the second array 12, may contain a single component from the set suspended at a known concentration. In at least some example embodiments, containers supplying liquid dispensers in one array, such as the first array 11, may carry liquids comprising of components from the set, but containers supplying liquid dispensers in the other array, such as at least the second array 12, may carry liquids which do not carry components. Such and example may be used, for example, wherein a pre-determined landing coordinate may be realised for each of the carrier drops 10 (10a, 10b...) in the stream 33 based on pre-calculated sums of momentums of drops during all moments of collisions between the carrier drop 10 and at least the drops that aggregately comprise the subset of components, or for example wherein a desired lower concentration of components in the carrier drop 10 may be achieved by colliding the carrier drop 10 with drops that carry no components. In at least some examples, the binding reagents used for assembling and binding together the components (in the correct order) are dispensed in the carrier drop 10 or a drop that collides with the carrier drop 10 before the carrier drop 10 lands on the drop collecting element 23. In at least some examples, drops that comprise no components collide with the carrier drop 10 during flight, and liquids may be added to the carrier drop 10 in this way, so that the carrier drop 10 does not evaporate before landing. In at least some example embodiments, at least one liquid dispenser in the plurality 44 of liquid dispenser may be used for the purpose of redundancy or any other similar purpose.

In the example embodiment of the device 1 as depicted in Figure 2, the first array 11 and the second array 12 are arranged so that nozzles from the liquid dispenser A2 and the liquid dispenser A6 are facing each other and are positioned at the same height, nozzles from the liquid dispenser A3 and the liquid dispenser A7 are facing each other and are positioned at the same height, nozzles from the liquid dispenser A4 and the liquid dispenser A8 are facing each other and are positioned at the same height, nozzles from the liquid dispenser A6 and the liquid dispenser A9 are facing each other and are positioned at the same heigh In the example embodiment of the device 1, such as depicted in Figure 2, the distance between two neighbouring nozzles in the first array 11 and the distance between two neighbouring nozzles in the second array 12 is a same. In other examples of the device 1 and the method 100 according to the present disclosure, such distance between various nozzles in the plurality 44 of liquid dispensers may not be the same and may be different. In other examples of the device 1 and the method 100 according to the present disclosure, the nozzles in the first array 11 and the nozzles in the second array 12 may be positioned at different heights. It is understood that various appropriate distances between any nozzles in the plurality 44 of liquid dispensers may be used in at least some example embodiments of the device 1 and the method 100 according to the present disclosure.

In at least some example embodiments, drops dispensed from the first array 11 and the second array 12 arrive at and collide with the carrier drop10 simultaneously. In the case wherein the drops dispensed from the first array 11 and the second array 12 are equal in size and arrive simultaneously from both sides to the carrier drop10 in the centre, the momentums of such drops dispensed from the first array 11 and the second array 12 cancel each other out at the moment of colliding with the carrier drop 10, and in this way, the carrier drop 10 remains in a straight forward trajectory towards the drop collecting element 23 after colliding with drops dispensed from the first array 11 and the second array 12.

In at least some example embodiments, drops dispensed from the first array 11 and the second array 12 arrive and collide with the carrier drop 10 simultaneously, but the drop dispensed from the first array 11 is not equal in size to the drop dispensed from the second array 12, and this causes a slight change in the trajectory of the carrier drop10 after the collision between the carrier drop 10 and the drops dispensed from the first array 11 and the second array 12. The slight change in the path of the carrier drop 10 may be pre-calculated and controlled by the control unit 100 so that each carrier drop 10 may arrive on the drop collecting element 23 at the pre-determined landing coordinate, wherein the pre-determined landing coordinate for each of the carrier drops 10 (10a, 10b...) may be next to the pre-determined landing coordinate of any other carrier drops 10 (10a, 10b...) in the stream 33 that land on the drop collecting element 23.

In at least some example embodiments, the device 1 may comprise of, for example, eighty (80) liquid dispensers arranged in the first array 11. In another example embodiment of the device 1, the device may comprise eighty (80) liquid dispensers in the first array 11 and eighty (80) liquid dispensers in at least the second array 12, and eighty (80) individual containers may be connected to eighty (80) nozzles in the first array 11, and another eighty (80) individual containers may be connected to eighty (80) nozzles in the second array 12. In a preferred example embodiment of the device 1, the device 1 comprises, for example, eighty (80) liquid dispensers per array, and eighty (80) individual containers may be connected to eighty (80) nozzles in the first array 11 array 11 and eighty (80) nozzles in the second array 12 at the same time. In another example embodiment of the device 1, the device 1 comprises, for example, eighty (80) liquid dispensers per array, eighty (80) individual containers may be connected to eighty (80) liquid dispensers in the first array 11, and a single container may be connected to all of the eighty (80) liquid dispensers in the second array 12.

In at least some example embodiments, the device comprises the control unit 1000 being configured to encode and map the data to be recorded in nucleic acids. The encoding and mapping, by using the control unit 100, shall be discussed hereinafter in more detail. Data may be either in a form of digital data or analogue data. In the case wherein the data is analogue data, the analogue data is firstly converted to digital data. Digital data is usually in the form of a string of binary symbols (0 and 1). During the encoding, the control unit 1000 is configured to transcode the string of binary symbols to a string of symbols having *n* possible values, wherein the transcoding depends on the example embodiment of the device 1 and the method 10 which is used with the device 1. In at least some examples of the encoding, the *n* value is four (4) so that each out of the four (4) values may correspond to each out of four (4) naturally occurring nucleic acid bases, which may be used to represent data in each of the components in the set. In at least some examples of the encoding, the *n* value is any possible value, which is suitable to represent digital data in the components from the set. It is understood that during the encoding, an encoding algorithm may be used, wherein the encoding algorithm includes all mathematical functions and operations performed in order to convert (or transcode) the string of binary symbols to the string of symbols having n possible values, based on parameters of the plurality of data segments available in the set and parameters of the data, so that during the encoding, the encoding algorithm abides by: a length of the data segments available in the set, a number of the data segments available in the set, *n* number of different symbols which are used to represent data, number of bits and bytes used in the representation of digital data, and so on. In this way, during the encoding, the digital data is converted (or in a more correct word: transcoded) to the string of symbols having *n* possible values based on the parameters of the data segments available in the set and the parameters of the digital data, by using the encoding algorithm. The control unit 1000 may, in the next step, map the string of distinct symbols haying *n* possible values, which was generated during the encoding, onto a plurality of successive data segments available in the set, so that each subsegment of the string of symbols haying *n* possible values is mapped onto one data segment available in the set. In this way, only a subset of data segments available in the set are chosen to represent the digital data, which was encoded, so that data segments in the plurality of successive data segments, which are mapped to represent digital data, are known. After the string of symbols having *n* possible values is mapped onto the plurality of successive data segments available in the set, the subset of components may be selected- Components from the subset, which is selected to represent data, aggregately comprise the data segments from the plurality of successive data segments, which represent data, and all correct gluing segments, which enable said data segments to be positioned in the correct order or indexed correctly. Mapping of the string of symbols having *n* possible values onto to the plurality of successive data segments, which are available in the set, may be based on the at least one look-up table. The look-up table may include information on the length of the data segments available in the set, the number of data segments available in the set, indexing information (including the information on the position of each component relative to any other component in the subset), encoding information, encoding algorithm, and other relevant information. It is understood to those skilled in the art that the information on the encoding and mapping steps as well as in the look up table, may also be recorded and represented in the subset, so that retrieval and reading back of the data, which is represented by the subset of components, may be performed in an efficient way.

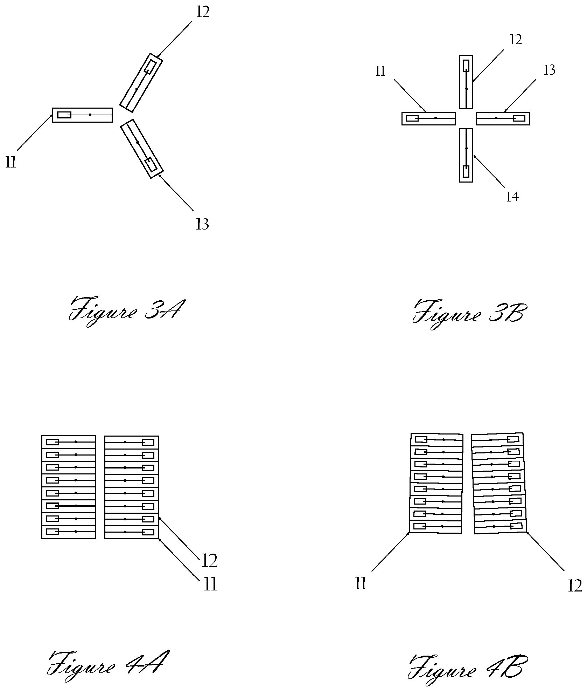

In at least some example embodiments of the device 1 and the method 100 according to the present disclosure, the device 1 and the method 100 comprise at least some liquid dispensers arranged in three (3) linear arrays of liquid dispensers, such as the first array 11, the second array 12 and a third array 13, as depicted schematically in a top view in Figure 3A.

In at least some example embodiments of the device 1 and the method 100 according to the present disclosure, the device 1 and the method 100 comprise at least some liquid dispensers arranged in four (4) linear arrays of liquid dispensers, the first array 11, the second array 12, a third array 13, and a four array 14, as depicted schematically in a top view in Figure 3B.

Figure 4A schematically depicts a top view of a plurality of linear arrays of liquid dispensers which are stacked next to each other. It is understandable that an example embodiment of the device 1 and the method 100 according to the present disclosure is possible wherein the device 1 includes a plurality of arrays (including at least the first array 11 and the second array 12) of liquid dispensers stacked next to each other, wherein said plurality of arrays of liquid dispensers may be used simultaneously. It is understandable that in such an example embodiment where the plurality of arrays of liquid dispensers are used, any number and combination of containers may supply liquids to any number and combination of nozzles in each array within any of the plurality of arrays in the device 1. Such a combination of liquids supply from a plurality of containers to the plurality of arrays and its individual dispensers may be realised by connecting a plurality of capillaries in a logical manner as appropriate. In order to control the supply of the plurality of liquids to the plurality of arrays in use, additional valves, gates, or other piezo, optical, acoustic, vibrational, thermic or other micro-fluid elements may be installed in such a liquid supply system. A preferred example embodiment of the device 1 and method 100 hereof may contain the plurality of arrays of liquid dispensers stacked next to each other, and a single container may supply liquids to all liquid dispensers in the plurality 44 that are configured to dispense the carrier drops 10 (10a, 10b...) in the device 1.

In at least some example embodiments of the device 1 and the method 100 according to the present disclosure, the first array 11 and the second array 12 are positioned vertically, but there is a slight vertical angle between the first array 11 and the second array 12, so that the upper parts of the first array 11 and the second array 12 are closer than the bottom parts of the first array 11 and the second array 12, as shown in a front view schematically in Figure 4B. As an example, such a vertical angle may be used for the purpose of avoiding contamination of the nozzles by the carrier drop 10. As the carrier drop 10 is becoming larger due to the sequential collision with other dispensed drops during the flight to the drop collecting element 23, a slight vertical angle between the first array 11 and the second array 12 may compensate for the enlargement of the carrier drop 10 volume in-flight due to the collision and consequently addition of the drops to the carrier drop 10.

In at least some example embodiments of the device 1, such as depicted in Figure 6, the drop collecting element 23 may be a larger container filled with liquid,. In such an example embodiment, a submersion element may be positioned between the plurality 44 of liquid dispenser and the drop collecting element 23 for the purpose of aiding the collection or submersion of the carrier drops 10 (10a, 10b...) in the liquid in the larger container. The submersion element may include a belt mechanisation such as schematically depicted in Figure 6, the belt or roller of which may be partially immersed in the liquid held by the drop collecting element 23, which is in this case the larger container.

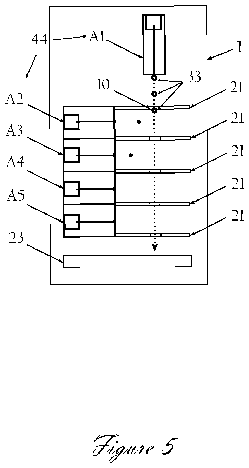

Figure 5 depicts an example embodiment of the device 1 and the method 100 according to the present disclosure wherein the trajectory of the carrier drops 10 (10a, 10b...) in the stream 33 are controlled with the use of electric fields. In such an example embodiment of the device 1 and the method 100 according to the present disclosure as depicted in Figure 5, the device 1 comprises electric plates 21 arranged perpendicularly to a path of at least one drop, such as the carrier drop 10, dispensed from at least one liquid dispenser, such as the liquid dispenser A1 in Figure 5, wherein the electric plates 21 have holes through which the at least one drop passes, the at least one liquid dispenser is configured to provide an electric charge to the at least one drop, and the electric plates 21 are configured so that the electric plates 21 exert electric fields on the at least one drop in a synchronised manner, so that the at least one drop is manipulated with the electric fields exerted by the electric plates 21 during a time in which the at least one drop passes through the electric fields exerted by the electric plates 21. The electric plates 21 have tiny holes through which the stream 33 of carrier drops 10 (10a, 10b...) passes. In one example, the electric plates 21 may comprise a multitude of electrodes in the shape of rings positioned around the holes in the electric plates 21. The electric plates 21 or electrodes may be electrically charged so so that electrical charge is accumulated in the electric plates 21 or in any of the electrodes in the multitude of electrodes so that a desired electric field is achieved. The electric plates 21 are configured so that the time-pattern of charging or discharging the electric plates 21 or the multitude of electrodes (and thus the presence of the desired electric field) are tightly synchronized with the stream 33 of carrier drops 10 (10a, 10b...) in order to achieve a desired effect wherein the desired effect is that of: stabilisation of the carrier drop 10 trajectory,, adjustment the carrier drop 10 trajectory, acceleration the carrier drop 10 trajectory, deceleration the carrier drop 10 trajectory, or any other manipulation of the carrier drop 10 trajectory. The carrier drop 10 trajectory may in this way be stabilised, or adjusted, or the carrier drops10 may be accelerated or decelerated. Repeatability of the stream 33 of carrier drops 10 (10a, 10b...) may thus be achieved so that the manipulation of the carrier drop 10 trajectory using electric fields exerted from the electric plates 21 enables compensation of the effects of collisions of the carrier drops 10 (10a, 10b...) with other the charge-free drops which collide with the carrier drop 10. In at least one example, each electric plate 21 is made of uniform conductive material. In at least another example, each of electric plates 21 is made of three (3) layers of materials: a layer of insulating material stacked between two layers of conductive material, and in such a case, the two conductive plates may be charged with opposite electrical charges which effectively form an electrical dipole. It is understood that depending on the charge of the carrier drops 10 (10a,10b...) and the desired effect of the electric fields exerted from the electric plates 21 on the carrier drop 10.voltage may either increase between two neighbouring electric plates 21 in the direction of the carrier drop 10 trajectory, or voltage may decrease between two neighbouring electric plates 21 in the direction of the carrier drop 10 trajectory. In this way, in at least some example embodiments of the device 1 and the method 100 according to the present disclosure, the trajectory of each of the carrier drops 10 (10a, 10b...) in the stream 33 may be manipulated by using the electric fields exerted by the electric plates 21 which are mounted perpendicularly to the carrier drop 10 trajectory and have holes through which the carrier drops 10 (10a, 10b...) pass. Electric plates 21 are controlled and configured by the control unit 1000, or by any peripheral control unit dedicated to such control. In another preferred example of the device 1 and the method 100 according to the present disclosure, magnetic fields may be used for the manipulation of trajectories of each carrier drop 10 in the stream 33. Similarly than in the above-mentioned example wherein electric fields are used for the purpose of carrier drop 10 trajectory manipulation, magnetic fields may also be used in some example embodiments of the device 1 and the method 100 according to the present disclosure. In such a case, magnetic field sources are mounted around the stream 33 of carrier drops 10 (10a,10b...) so that an appropriate magnetic field which may be used to manipulate the trajectories of carrier drops 10 (10a, 10b...) in the stream 33 is produced. In one example, the magnetic field sources may be permanent magnets, and may be mounted in dipole, quadrupole or other forms to produce suitable magnetic fields. In another example, the magnetic field sources may be electromagnets in the shape of electric coils (such as but not limited to: 2 phase windings such as a solenoid; 3 phase windings, other), and may be mounted in a way to produce a dipole magnetic field, quadrupole magnetic field, or other shapes of magnetic fields. Magnetic field sources may be controlled via the command signals from the control unit 1000 or via any peripheral control unit dedicated to such control. It is understood to those skilled in the art, that alternating or permanent magnetic fields may be produced as necessary, depending on the magnetic properties of the liquids used in the carrier drops 10 (10a, 10b...).

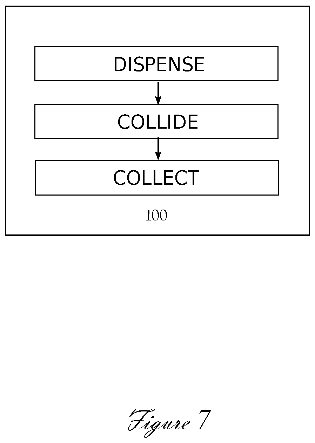

Hereinafter some example embodiments that relate to the method 100 for recording data in nucleic acids are described in more detail. Main steps of the method 100 are schematically depicted in Figure 7. The method 100 for recording data in nucleic acids (hereinafter: the method 100) comprises at least dispensing, by using a plurality of liquid dispensers A1-A3, at least one carrier drop 10 and at least drops that aggregately comprise a subset of components from a set of components, wherein the carrier drop 10 sequentially collides with at least drops that aggregately comprise the subset of components, so that the subset of components is located in the carrier drop 10 during flight and represents data, and collecting the carrier drop 10 by using a drop collecting element 23.

In at least some example embodiments, the method 100 comprises pre-preparing the set of nucleic acid components in a way so that gluing segments are added to data segments of components. Each distinct component from the set may therefore contain a data segment which carries information, and at least one gluing segment which enables the component to bind to another component and may carry indexing information describing a position of the component to which the gluing segment is bound to, relative to other components in the subset.

In at least some example embodiments, the method 100 comprises providing user data to the device 1 by using a user interface. After the user data have been provided the user may give a signal to start the device 1. In at least some example methods of device 1 operation, after the user data is provided by the user, the user data may be processed by a control unit 1000 (Fig. 2), and all of the necessary control signals for each and every part of the device 1 for the purpose of executing all of the operational steps the device 1 which will result in the data being recorded in the nucleic acids may be generated by the control unit 1000. In some example embodiments, a software part of the control unit 1000 may be located at another location so that the pre-processing of control signals might be generated at a location, which is different than a location of the device 1. This enables the user to produce control signals for the device 1 prior to being physically near the device 1.

In at least some example embodiments, the method 100 may include dispensing, by using at least one liquid dispenser in the plurality 44 of liquid dispensers, the carrier drop 10 that has a trajectory that passes by at least two liquid dispensers in the device 1 that are configured to dispense drops towards the trajectory of the carrier drop 10 in a synchronised manner, and colliding the carrier drop 10 sequentially with at least all of the drops that aggregately comprise components from the subset and are dispensed from the at least two liquid dispensers.

In at least some example embodiments, the method 100 may include dispensing a stream 33 (Fig. 1) of carrier drops 10 (10a, 10b...) by using at least one liquid dispenser in the plurality 44, wherein the stream 33 may include a first carrier drop 10, a second carrier drop 10a, and at least a third carrier drop 10b. In at least some example embodiments, the method 100 may include colliding, by using the plurality 44 of liquid dispensers, the first carrier drop 10 with at least drops that aggregately comprise components from a first subset, the second carrier drop 10a with at least drops that aggregately comprise components from a second subset, the third carrier drop 10b with at least drops that aggregately comprise components from a third subset, and so on, until all of the carrier drops 10 (10a, 10b...) in the stream 33 collide and merge with at least some drops that aggregately comprise at least some components from the set, which represent data; and collecting, by using the drop collecting element 23, all of the carrier drops 10 (10a, 10b ... ) in the stream 33.

In one example embodiment, the method 100 may include arranging two or more liquid dispenser in an array, such as a linear array, a circular array, a mesh array, or any other suitable type of array comprising at least two liquid dispensers arranged in an ordered manner. In one example embodiment, the method 100 may include arranging at least some liquid dispensers in at least a first array 11 and a second array 12 (Fig. 2), and dispensing drops, by using at least the first array 11 and the second array 12, towards a single stream 33 of carrier drops 10 (10a, 10b...). In one example embodiment, the method 100 may include dispensing drops, by using the first array 11, towards a first stream 33 of carrier drops 10 (10a,10b...), and dispensing drops, by using the second array 12, toward a second stream 33 of carrier drops 10 (10a, 10b...).

In one example embodiment, the method 100 may include dispensing, by using each of the liquid dispensers in the plurality 44 of liquid dispensers, up to a hundred thousand (100000) drops per second. In one example embodiment, the method 100 may include dispensing, by using each of the liquid dispensers in the plurality 44 of liquid dispensers, drops with a volume in a range between one (1) picoliter to a hundred (100) nanoliters. In one example embodiment, the method 100 may include dispensing drops, by using the plurality 44 of liquid dispensers including fifty or more, or five hundred or more, or ten thousands or more liquid dispensers.

In at least some example embodiments, the method 100 may include collecting the carrier drops 10 (10a, 10b...) by using the drop collecting element 23 made of either a bendable material or a non-bendable material. In at least some example embodiments, the method 100 may include collecting the carrier drops 10 (10a, 10b...)by using the drop collecting element 23, which is either detachable or non-detachable relative to the device 1. In at least some example embodiments, the method 100 may include collecting the carrier drops 10 (10a, 10b...)by using the drop collecting element 23, which is a container filled with a liquid in which the carrier drops land. In at least some example embodiments, the method 100 may include collecting the carrier drops 10 (10a,10b...)by using the drop collecting element 23, which is a surface that may be flat.

In at least some example embodiments, the method 100 may include dispensing drops of a first liquid by using the plurality 44 of liquid dispensers, and filling or covering the drop collecting element 23 with a second liquid, and the first liquid and the second liquid are immiscible.

In at least some example embodiments, the method 100 may include arranging electric plates 21 perpendicularity to a path of at least one drop dispensed from at least one liquid dispenser, wherein the electric plates 21 have holes through which the at least one drop passes, electrically charging, by using the al least one liquid dispenser, the at least one drop, and manipulating the at least one drop by exerting electric fields onto the at least one drop by using the electric plates 21 in a synchronised manner, so that the at least one drop is manipulated by the electric fields exerted by the electric plates 21 during a time in which the at least one drop passes through the electric fields exerted by the electric plates 21.

In at least some example embodiments, the method 100 may include positioning the plurality 44 of liquid dispensers and the drop collecting element 23 in a mixing chamber 50, which is a partially or a fully closed compartment of the device 1. In one example, the method 100 may include closing the mixing chamber 50 during the time in which the drops are in-flight, so that the mixing chamber 50 is the fully closed compartment during the time in which the drops are in-flight.

In at least some example embodiments, the method 100 may include dispensing, by using at least one liquid dispenser in the plurality 44 of liquid dispensers, the carrier drop 10 that may include binding reagents, which enable an assembly and binding of the components located in the carrier drop 10. In at least some example embodiments, the method 100 may include dispensing, by using at least one liquid dispenser in the plurality 44 of liquid dispensers, a drop that may include the binding reagents and collides and merges with the carrier drop 10 during flight.

The method 100 includes achieving, by using a temperature regulator 401, a sequence of temperatures in a processing chamber 60 over time, and causing, by the sequence of temperatures, a biochemical reaction to occur between the components and binding reagents located in each carrier drop 10 positioned in the processing chamber 60, so that the components located in the each carrier drop 10 positioned in the processing chamber 60 assemble and bind together to form final nucleic acid molecules, which represent data. The biochemical reaction, which is used for assembling and binding components together may be any appropriate biochemical reaction that results in the assembling and binding together of the components, such as:a Polymerase Chain Reaction,a Sticky End Ligation, a Cell Free Cloning, a BioBricks Assembly, a Gibson Assembly, a HiFi Assembly, and wherein at least some of the biochemical reactions include multiplication of the final nucleic acid molecules.

In at least some example embodiments, the method 100 may include moving, by using a mechanisation 22, the drop collecting element 23 in at least one direction relative to the plurality 44 of liquid dispensers. In at least some example embodiments, the method 100 may include moving, by using the mechanisation 22, the plurality 44 of liquid dispensers in at least one direction relative to the drop collecting element 23. In at least some example embodiments, the method 100 may include moving, by using the mechanisation 22, the drop collecting element 23 from the mixing chamber 50 to the processing chamber 60.

In at least some example embodiments, the method 100 may include: holding a liquid, by using a container, in each of the liquid dispensers in the plurality 44 of liquid dispensers, connecting the container, by using a capillary, to a nozzle in the each of the liquid dispensers, dispensing, by using a drop generating element, a drop in the each of the liquid dispensers, so that the liquid runs from the container through the capillary and out through the nozzle and the drop is dispensed in this way; and operating, by using a synchronisation means in the each of the liquid dispensers, the plurality 44 of liquid dispensers in a synchronised manner. In at least some example embodiments, the method 100 may include supplying one liquid, by using a single container, to two or more nozzles in the plurality 44 of liquid dispensers. In at least some example embodiments, the method 100 may include supplying two or more liquids, by using two or more containers, to a single nozzle in the plurality 44 of liquid dispensers.

In at least some example embodiments, the method 100 may include encoding and mapping, by using a control unit 100, data to be recorded in nucleic acids. In at least some example embodiments, the method 100 may include selecting, by using the control unit 100, the subset of components from the set, so that the subset of components represents data. In at least some example embodiments of the method 100, the method 100 may include using any of the devices 1 disclosed herein.

In at least some preferred example embodiments of the device 1 and the method 100 according to the present disclosure, the device 1 and the method 100 comprise two hundred and fifty-six (256) liquid dispensers arranged in the first array 11, and one liquid dispenser configured to produce the stream of carrier drops. In another preferred example embodiment of the device 1 and the method 100, the device 1 and the method 100 comprise two hundred and fifty-six (256) liquid dispensers arranged in the first array 11, two hundred and fifty-six (256) liquid dispensers arranged in the second array 12, one liquid dispenser configured to produce the first stream 33 of carrier drops10 (10a, 10b...) which collides with the drops dispensed from the first array 11, and at least one liquid dispenser is configured to produce the second stream 33 of carrier drops 10 (10a, 10b...) that collide with the drops dispensed from the second array 12.

Hereinafter one example of recording data in nucleic acids by using the device 1 and the method 100 is described in more detail.

Components may be oligonucleotides. The set may be purchased or pre-prepared and may contain certain permutations or variations of oligonucleotides. In one example embodiment, the set may be pre-prepared so that at least eight (8) unique gluing segments are bound onto the beginnings or endings of ten (10) uniquely coded deoxyribonucleotide sequences which represent ten (10) available data segments in the set. Each of the at least eight (8) unique gluing segments enables each of the ten (10) available data segments to be indexed or positioned at a specific position out of eight (8) possible positions in the subset or the final nucleic acids. In this given example, the subset of components that represents data or the final nucleic acid components that represent data comprise eight (8) components in the correct order, and (8) possible positions within the subset of the final nucleic acids are available and provided by the at least eight (8) unique gluing segments. When the set is pre-prepared in this way, the set may comprise eighty (80) components, wherein each component comprises one data segment and at least one gluing segment. Each component out of the eighty (80) components in the set, which may be pre-prepared in this way, is suspended at a known concentration in a liquid which may be water or similar aqueous solution capable of carrying components, and is held in one container supplying the liquid to at least on liquid dispenser in the device 1. Liquids which carry such pre-prepared components from the set are placed into the plurality of containers which supply the at least some liquid dispensers in the plurality 44 of liquid dispensers in the device. Each container in the device 1 which supplies any of the liquid dispensers which is configured to dispense drops which include components from the set, holds a known concentration of predefined and precast components suspended in an appropriate liquid.

The control unit 1000 may be configured to control one liquid dispenser, such as the liquid dispenser 1A, to dispense the carrier drop 10 on demand at an exact moment in time. The control unit 1000 may be configured to control the plurality 44 of liquid dispensers, such as, for example the liquid dispensers A2-A9 in Fig. 2, to dispense drops that include components, at exact moments in time.

As mentioned previously and in this given example, the set may be pre-prepared out of ten (10) distinct data segments specifically hybridized via the complementary sequences of at least eight (8) distinct gluing segments which allow any of the ten (10) data segments to be positioned at any place within, in the given example, eight (8) possible places in the subset or in the final nucleic acid molecules. The set of eighty (80) components may be pre-prepared in this way. In the case wherein the final nucleic acid molecule comprises of eight (8) components assembled and bound together in the specific order, data is therefore represented by the subset comprising eight (8) components selected from the set of eighty (80) components.

The control unit 1000 may select the subset of eight (8) components so that the components represent digital data, and controls the eight (8) liquid dispensers which dispense drops which include the eight (8) components from the subset, so that the carrier drop 10 sequentially collides with all of the eight (8) drops which include all of the eight (8) components.

The trajectory of the carrier drop 10 and the trajectory of the drops which include components from the subset cross at an exactly the right time and at an exactly the right position so that the carrier drop 10 sequentially collides with all of the drops which include all eight (8) components from the subset, and all of the eight (8) components from the subset are collected by the carrier drop 10 before the carrier drop 10 lands on the drop collecting element 23. In this example, to synthesize the final nucleic acid molecules which include eight (8) components bound in the correct order, out of eighty (80) liquid dispensers available, the plurality of eight (8) liquid dispensers will dispense drops that include components from the subset and one liquid dispenser, such as the liquid dispenser A1, wdispenses the carrier drop 10. In this way, the correct number and combination of drops which include components from the subset is dispensed, collided and merged with the carrier drop 10. After all sequential collisions between the carrier drop 10 and the at least eight (8) drops which include all wight (8) components occurs, and when the eight (8) selected components in the subset (out of the set of eighty (80) components) are located in the carrier drop 10, the carrier drop 10 lands on the drop collecting element 23. In at least some example embodiments, the binding reagents are included in the carrier drop 10 or in the drop that collides with the carrier drop 10, so that the binding reagents which may include a standard PCR Master Mix fluid, are located in the carrier drop 10 together with the eight (8) components. It is understandable that the stream 33 of carrier drops 10 (10a, 10b...) may be dispensed and that the control unit 1000 may select a plurality of subsets and control the plurality 44 of liquid dispensers, so that each carrier drop 10 in the stream 33 may collect a distinct subset from the plurality of subsets before landing on the drop collecting element 23. After the carrier drops 10 (10a, 10b...) land on the drop collecting element 23, the control unit 1000 may be configured to control the mechanisation 22 so that the mechanisation 22 moves the drop collecting element 23 from the mixing chamber 50 to the processing chamber 60. When the carrier drops 10 (10a, 10b...) are located in the processing chamber 60, the temperature regulator 402 is configured to provide the sequence of temperatures so that the PCR occurs in the carrier drops 10 (10a,10b...). Because the plurality of carrier drops 10 (10a,10b...) may be located on the drop collecting element 23, the PCR may occur simultaneously in the plurality of carrier drops 10 (10a, 10b...) which are located the drop collecting element 23, which is positioned in the processing chamber 60. In at least one example embodiment wherein the components are suspended in water or buffer with specific pH or any other water-soluble liquid for storing components, the drop collecting element 23 may contain or may be covered with hydrophobic liquid so that the plurality of carrier drops 10 (10a, 10b...) may remain intact and suspended in hydrophobic liquid after landing on/in the drop collecting element 23, and the PCR may occur simultaneously in the plurality of carrier drops 10 (10a, 10b...) located in the processing chamber 60 without cross-over contamination of liquids between the carrier drops 10 (10a, 10b...). After the PCR occurs successfully in the carrier drops 10 (10a, 10b...) located in the processing chamber 60, the final nucleic acid molecules that represent data are produced and may be removed from the device 1. In this way, data may be recorded in the final nucleic acid molecules by using the device 1 and the method 100 according to the present disclosure.

## Claims

1. A device (1) for recording data in nucleic acids, comprising:
- a plurality of liquid dispensers (A1-A9) which comprises a liquid dispenser (A1) configured to dispense a carrier drop (10),
- a drop collecting element (23) configured to collect the carrier drop (10), wherein during operation of the device (1) the carrier drop (10) flies from the liquid dispenser (A1) towards the drop collecting element (23) in a trajectory, and
- at least two other liquid dispensers (A2, A3) of the plurality of liquid dispensers (A1-A9), arranged such that the carrier drop's trajectory passes by the at least two other liquid dispensers (A2, A3), wherein the at least two other liquid dispensers (A2, A3) are configured to dispense two respective drops towards the trajectory of the carrier drop (10) in a synchronised manner, such that the carrier drop (10) sequentially collides with the two drops, wherein each drop of the two drops aggregately comprises a subset of components from a set of nucleic acid components, thereby the subsets of components, representing data, are located in the carrier drop (10) during flight and before the carrier drop lands on the drop collecting element (23),
wherein the device (1) comprises a processing chamber (60) having at least one temperature regulator (402) configured to change a temperature in the processing chamber (60) over time so that a sequence of temperatures is achieved over time in the processing chamber (60), and the sequence of temperatures causes a biochemical reaction to occur between the components and binding reagents located in each carrier drop (10) positioned in the processing chamber (60), wherein the biochemical reaction causes the components located in each of the carrier drops (10) to assemble and bind together to form final nucleic acid molecules, which represent data,
wherein the biochemical reaction is a Polymerase Chain Reaction, a Sticky End Ligation, a Cell Free Cloning, a BioBricks Assembly, a Gibson Assembly, or a HiFi Assembly, and wherein at least some of the biochemical reactions include multiplication of the final nucleic acid molecules.

2. A device (1) according to claim 1, **characterised in that** at least one liquid dispenser (A1) is configured to dispense a stream (33) of carrier drops (10), wherein the stream (33) comprises a first carrier drop (10), a second carrier drop (10a), and at least a third carrier drop (10b), and the plurality of liquid dispensers (A1-A9) are configured so that the first carrier drop (10) collides with at least drops that aggregately comprise components from a first subset, the second carrier drop (10a) collides with at least drops that aggregately comprise components from a second subset, the third carrier drop (10b) collides with at least drops that aggregately comprise components from a third subset, and so on, until all of the carrier drops in the stream (33) collide with at least some drops that aggregately comprise at least some components from the set, and all of the carrier drops (10, 10a, 10b) in the stream (33) land on the drop collecting element (23).

3. A device (1) according to any one of the preceding claims, **characterised in that** the two or more liquid dispensers (A2-A5, A6-A9) in the plurality of liquid dispensers (A1-A9) are arranged in an array (11-14), such as a linear array or a circular array..

4. A device (1) according to claim 1 or 2, wherein at least some of the liquid dispensers are arranged in at least a first array (11-14) and a second array (11-14), wherein the first array (11-14) and the second array (11-14) are configured to dispense drops towards a single stream (33) of the carrier drops (10, 10a, 10b), or the first array (11-14) is configured to dispense drops towards a first stream (33) of carrier drops (10, 10a, 10b), and the second array (11-14) is configured to dispense drops towards a second stream (33) of carrier drops (10, 10a, 10b).

5. A device (1) according to any one of the preceding claims, **characterised in that** each of the liquid dispensers in the plurality of liquid dispensers (A1-A9) is configured to dispense up to a hundred thousand (e.g. 100000) drops per second, and/or is configured to dispense drops with a volume in a range between one picoliter (e.g. 1 pl) to a hundred nanoliters (e.g. 100 nl); and/or the plurality of liquid dispensers (A1-A9) comprises fifty or more, or five hundred or more, or ten thousands or more liquid dispensers.

6. A device (1) according to any one of the preceding claims, **characterised in that** the drop collecting element (23) is made of a bendable material or a non-bendable material, and/or is detachable or non-detachable relative to the device (1).

7. A device (1) according to any one of the preceding claims, wherein the drop collecting element (23) is a container filled with a liquid in which the carrier drops (10) land, and/or is a surface.

8. A device (1) according to any one of the preceding claims, **characterised in that** the plurality of liquid dispensers (A1-A9) are configured to dispense drops of a first liquid, and the drop collecting element (23) is filled or covered with a second liquid, and the first liquid and the second liquid are immiscible.

9. A device (1) according to any one of the preceding claims, **characterised in that** the device (1) comprises electric plates (21) arranged perpendicularly to the path of at least one drop dispensed from at least one liquid dispenser (A1), wherein the electric plates (21) have holes through which the at least one drop passes, the at least one liquid dispenser (A1) is configured to provide an electric charge to the at least one drop, and the electric plates (21) are configured so that the electric plates (21)exert electric fields on the at least one drop in a synchronised manner, so that the at least one drop is manipulated with the electric fields exerted by the electric plates (21) during the time in which the at least one drop passes through the electric fields exerted by the electric plates (21).

10. A device (1) according to any one of the preceding claims, **characterised in that** the device (1) comprises a mixing chamber (50), which is a partially or a fully closed compartment of the device (1) wherein the plurality of liquid dispensers (A1-A9) and at least one drop collecting element (23) are positioned, and the mixing chamber (50) is the fully closed compartment during the time in which the drops are in-flight.

11. A device (1) according to any one of the preceding claims, **characterised in that** the carrier drop (10) includes binding reagents, which enable an assembly and binding of the components located in the carrier drop (10), wherein the binding reagents are included in the carrier drop (10) or provided by a drop that collides and merges with the carrier drop (10) during flight.

12. A device (1) according to any one of the preceding claims, **characterised in that** the device (1) comprises a mechanisation (22) configured to perform any of or any combination of the following:
- move the drop collecting element (23) in at least one direction relative to the plurality of liquid dispensers (A1-A9),
- move the plurality of liquid dispensers (A1-A9) in at least one direction relative to the drop collecting element (23),
- move the drop collecting element (23) from the mixing chamber (50) to the processing chamber (60).

13. A device (1) according to any one of the preceding claims, **characterised in that** each of the liquid dispensers in the plurality of liquid dispensers (A1-A9) comprises at least a container for holding liquids to dispense, a drop generating element positioned and configured to cause at least one drop to dispense, a nozzle through which the at least one drop is ejected from, a capillary which connects the container with the nozzle so that the liquid runs from the container through the capillary and out through the nozzle for the at least one drop to be dispensed, and synchronisation means, which enable each of the liquid dispensers to operate in a synchronised manner with other liquid dispensers in the plurality of liquid dispensers (A1-A9); .

14. A device (1) according to any one of the preceding claims, wherein a single container is configured to supply liquids to two or more nozzles in the plurality of liquid dispensers (A1-A9); and/or two or more containers are configured to supply liquids to a single nozzle in the plurality of liquid dispensers (A1-A9).

15. A method (100) for recording data in nucleic acids, using a plurality of liquid dispensers (A1-A9) that comprises a liquid dispenser (A1), a drop collecting element (23), and at least two other liquid dispensers (A2, A3), the method (100) comprising:
- dispensing, by use of the liquid dispenser (A1), a carrier drop (10) which flies from the liquid dispenser (A1) towards the drop collecting element (23) in a trajectory, which passes by the at least two other liquid dispensers (A2, A3),
- dispensing, by use of at least two other liquid dispensers (A2, A3), two respective drops towards the trajectory of the carrier drop (10) in a synchronised manner, such that the carrier drop (10) sequentially collides with the two drops, wherein each drop of the two drops aggregately comprises a subset of components from a set of nucleic acid components, thereby the subsets of components, representing data, are located in the carrier drop (10) during flight and before the carrier drop (10) lands on the drop collecting element (23), which collects the carrier drop (10), and
- changing, by at least one temperature regulator (402), a temperature in a processing chamber (60) over time so that a sequence of temperatures is achieved over time in the processing chamber (60), and the sequence of temperatures causes a biochemical reaction to occur between the components and binding reagents located in each carrier drop (10) positioned in the processing chamber (60), wherein the biochemical reaction causes the components located in each of the carrier drops (10) to assemble and bind together to form final nucleic acid molecules, which represent data,
wherein the biochemical reaction is a Polymerase Chain Reaction, a Sticky End Ligation, a Cell Free Cloning, a BioBricks Assembly, a Gibson Assembly, or a HiFi Assembly, and wherein at least some of the biochemical reactions include multiplication of the final nucleic acid molecules.

## Patentansprüche

1. Vorrichtung (1) zum Aufzeichnen von Daten in Nukleinsäuren, umfassend:
- eine Vielzahl von Flüssigkeitsabgabeeinrichtungen (A1-A9), die eine Flüssigkeitsabgabeeinrichtung (A1) umfasst, die dazu konfiguriert ist, einen Trägertropfen (10) abzugeben,
- ein Tropfensammelelement (23), das dazu konfiguriert ist, den Trägertropfen (10) zu sammeln, wobei während eines Betriebs der Vorrichtung (1) der Trägertropfen (10) von der Flüssigkeitsabgabeeinrichtung (A1) in Richtung des Tropfensammelelements (23) in einer Flugbahn fliegt, und
- mindestens zwei andere Flüssigkeitsabgabeeinrichtungen (A2, A3) der Vielzahl von Flüssigkeitsabgabeeinrichtungen (A1-A9), die so angeordnet sind, dass die Flugbahn des Trägertropfens an den mindestens zwei anderen Flüssigkeitsabgabeeinrichtungen (A2, A3) vorbeiführt, wobei die mindestens zwei anderen Flüssigkeitsabgabeeinrichtungen (A2, A3) dazu konfiguriert sind, zwei jeweilige Tropfen in Richtung der Flugbahn des Trägertropfens (10) auf eine synchronisierte Weise abzugeben, sodass der Trägertropfen (10) nacheinander mit den zwei Tropfen kollidiert, wobei jeder Tropfen der zwei Tropfen aggregiert eine Teilmenge von Komponenten aus einer Menge von Nukleinsäurekomponenten umfasst, wodurch sich die Teilmengen von Komponenten, die Daten darstellen, während des Flugs und bevor der Trägertropfen auf dem Tropfensammelelement (23) landet, in dem Trägertropfen (10) befinden,
wobei die Vorrichtung (1) eine Verarbeitungskammer (60) umfasst, die mindestens einen Temperaturregler (402) aufweist, der dazu konfiguriert ist, eine Temperatur in der Verarbeitungskammer (60) im Laufe der Zeit zu ändern, sodass eine Temperaturabfolge im Laufe der Zeit in der Verarbeitungskammer (60) erreicht wird und die Temperaturabfolge bewirkt, dass eine biochemische Reaktion zwischen den Komponenten und Bindungsreagenzien auftritt, die sich in jedem Trägertropfen (10) befinden, der in der Verarbeitungskammer (60) positioniert ist, wobei die biochemische Reaktion bewirkt, dass die Komponenten, die sich in jedem der Trägertropfen (10) befinden, assemblieren und aneinanderbinden, um endgültige Nukleinsäuremoleküle zu bilden, die Daten darstellen,
wobei es sich bei der biochemischen Reaktion um eine Polymerase-Kettenreaktion, eine Sticky-End-Ligation, eine zellenfreie Klonierung, eine BioBricks-Assemblierung, eine Gibson-Assemblierung oder eine HiFi-Assemblierung handelt und wobei mindestens einige der biochemischen Reaktionen eine Vervielfältigung der endgültigen Nukleinsäuremoleküle beinhalten.

2. Vorrichtung (1) nach Anspruch 1, **dadurch gekennzeichnet, dass** mindestens eine Flüssigkeitsabgabeeinrichtung (A1) dazu konfiguriert ist, einen Strom (33) von Trägertropfen (10) abzugeben, wobei der Strom (33) einen ersten Trägertropfen (10), einen zweiten Trägertropfen (10a) und mindestens einen dritten Trägertropfen (10b) umfasst und die Vielzahl von Flüssigkeitsabgabeeinrichtungen (A1-A9) derart konfiguriert ist, dass der erste Trägertropfen (10) mit mindestens Tropfen kollidiert, die aggregiert Komponenten aus einer ersten Teilmenge umfassen, der zweite Trägertropfen (10a) mit mindestens Tropfen kollidiert, die aggregiert Komponenten aus einer zweiten Teilmenge umfassen, der dritte Trägertropfen (10b) mit mindestens Tropfen kollidiert, die aggregiert Komponenten aus einer dritten Teilmenge umfassen, und so weiter, bis sämtliche der Trägertropfen in dem Strom (33) mit mindestens einigen Tropfen kollidieren, die aggregiert mindestens einige Komponenten aus der Menge umfassen, und sämtliche der Trägertropfen (10, 10a, 10b) in dem Strom (33) auf dem Tropfensammelelement (23) landen.

3. Vorrichtung (1) nach einem der vorhergehenden Ansprüche, **dadurch gekennzeichnet, dass** die zwei oder mehreren Flüssigkeitsabgabeeinrichtungen (A2-A5, A6-A9) in der Vielzahl von Flüssigkeitsabgabeeinrichtungen (A1-A9) in einer Anordnung (11-14), beispielsweise einer linearen Anordnung oder einer kreisförmigen Anordnung, angeordnet sind.

4. Vorrichtung (1) nach Anspruch 1 oder 2, wobei mindestens einige der Flüssigkeitsabgabeeinrichtungen in mindestens einer ersten Anordnung (11-14) und einer zweiten Anordnung (11-14) angeordnet sind, wobei die erste Anordnung (11-14) und die zweite Anordnung (11-14) dazu konfiguriert sind, Tropfen in Richtung eines einzelnen Stroms (33) der Trägertropfen (10, 10a, 10b) abzugeben, oder die erste Anordnung (11-14) dazu konfiguriert ist, Tropfen in Richtung eines ersten Stroms (33) von Trägertropfen (10, 10a, 10b) abzugeben, und die zweite Anordnung (11-14) dazu konfiguriert ist, Tropfen in Richtung eines zweiten Stroms (33) von Trägertropfen (10, 10a, 10b) abzugeben.

5. Vorrichtung (1) nach einem der vorhergehenden Ansprüche, **dadurch gekennzeichnet, dass** jede der Flüssigkeitsabgabeeinrichtungen in der Vielzahl von Flüssigkeitsabgabeeinrichtungen (A1-A9) dazu konfiguriert ist, bis zu hunderttausend (z. B. 100.000) Tropfen pro Sekunde abzugeben, und/oder dazu konfiguriert ist, Tropfen mit einem Volumen in einem Bereich zwischen einem Pikoliter (z. B. 1 pi) und hundert Nanolitern (z. B. 100 nl) abzugeben; und/oder die Vielzahl von Flüssigkeitsabgabeeinrichtungen (A1-A9) fünfzig oder mehr oder fünfhundert oder mehr oder zehntausend oder mehr Flüssigkeitsabgabeeinrichtungen umfasst.

6. Vorrichtung (1) nach einem der vorhergehenden Ansprüche, **dadurch gekennzeichnet, dass** das Tropfensammelelement (23) aus einem biegbaren Material oder einem nicht biegbaren Material hergestellt ist und/oder relativ zu der Vorrichtung (1) abnehmbar oder nicht abnehmbar ist.

7. Vorrichtung (1) nach einem der vorhergehenden Ansprüche, wobei es sich bei dem Tropfensammelelement (23) um einen Behälter, der mit einer Flüssigkeit gefüllt ist, in dem die Trägertropfen (10) landen, und/oder eine Oberfläche handelt.

8. Vorrichtung (1) nach einem der vorhergehenden Ansprüche, **dadurch gekennzeichnet, dass** die Vielzahl von Flüssigkeitsabgabeeinrichtungen (A1-A9) dazu konfiguriert ist, Tropfen einer ersten Flüssigkeit abzugeben, und das Tropfensammelelement (23) mit einer zweiten Flüssigkeit gefüllt oder bedeckt ist und die erste Flüssigkeit und die zweite Flüssigkeit nicht mischbar sind.

9. Vorrichtung (1) nach einem der vorhergehenden Ansprüche, **dadurch gekennzeichnet, dass** die Vorrichtung (1) Elektroplatten (21) umfasst, die senkrecht zu dem Weg mindestens eines Tropfens, der aus mindestens einer Flüssigkeitsabgabeeinrichtung (A1) abgegeben wird, angeordnet sind, wobei die Elektroplatten (21) Löcher aufweisen, die der mindestens eine Tropfen durchläuft, die mindestens eine Flüssigkeitsabgabeeinrichtung (A1) dazu konfiguriert ist, dem mindestens einen Tropfen eine elektrische Ladung bereitzustellen, und die Elektroplatten (21) derart konfiguriert sind, dass die Elektroplatten (21) elektrische Felder auf den mindestens einen Tropfen auf eine synchronisierte Weise ausüben, sodass der mindestens eine Tropfen mit den elektrischen Feldern, die durch die Elektroplatten (21) ausgeübt werden, während der Zeit, in welcher der mindestens eine Tropfen die elektrischen Felder durchläuft, die durch die Elektroplatten (21) ausgeübt werden, manipuliert wird.

10. Vorrichtung (1) nach einem der vorhergehenden Ansprüche, **dadurch gekennzeichnet, dass** die Vorrichtung (1) eine Mischkammer (50) umfasst, bei der es sich um einen teilweise oder einen vollständig geschlossenen Raum der Vorrichtung (1) handelt, in dem die Vielzahl von Flüssigkeitsabgabeeinrichtungen (A1-A9) und mindestens ein Tropfensammelelement (23) positioniert sind, und es sich bei der Mischkammer (50) um den vollständig geschlossenen Raum während der Zeit handelt, in der die Tropfen im Flug sind.

11. Vorrichtung (1) nach einem der vorhergehenden Ansprüche, **dadurch gekennzeichnet, dass** der Trägertropfen (10) Bindungsreagenzien beinhaltet, die eine Assemblierung und Bindung der Komponenten ermöglichen, die sich in dem Trägertropfen (10) befinden, wobei die Bindungsreagenzien in dem Trägertropfen (10) beinhaltet sind oder durch einen Tropfen bereitgestellt werden, der während des Flugs mit dem Trägertropfen (10) kollidiert und verschmilzt.

12. Vorrichtung (1) nach einem der vorhergehenden Ansprüche, **dadurch gekennzeichnet, dass** die Vorrichtung (1) eine Mechanisierung (22) umfasst, die dazu konfiguriert ist, eines oder eine Kombination der Folgenden durchzuführen:
- Bewegen des Tropfensammelelements (23) in mindestens eine Richtung relativ zu der Vielzahl von Flüssigkeitsabgabeeinrichtungen (A1-A9),
- Bewegen der Vielzahl von Flüssigkeitsabgabeeinrichtungen (A1-A9) in mindestens eine Richtung relativ zu dem Tropfensammelelement (23),
- Bewegen des Tropfensammelelements (23) aus der Mischkammer (50) in die Verarbeitungskammer (60).

13. Vorrichtung (1) nach einem der vorhergehenden Ansprüche, **dadurch gekennzeichnet, dass** jede der Flüssigkeitsabgabeeinrichtungen in der Vielzahl von Flüssigkeitsabgabeeinrichtungen (A1-A9) mindestens einen Behälter zum Aufnehmen von abzugebenden Flüssigkeiten, ein Tropfenerzeugungselement, das dazu positioniert und konfiguriert ist, zu bewirken, dass mindestens ein Tropfen abgegeben wird, eine Düse, durch die der mindestens eine Tropfen ausgestoßen wird, eine Kapillare, die den Behälter mit der Düse verbindet, sodass die Flüssigkeit aus dem Behälter durch die Kapillare und durch die Düse herausläuft, damit der mindestens eine Tropfen abgegeben wird, und Synchronisationsmittel umfasst, die es jeder der Flüssigkeitsabgabeeinrichtungen ermöglichen, auf eine synchronisierte Weise mit anderen Flüssigkeitsabgabeeinrichtungen in der Vielzahl von Flüssigkeitsabgabeeinrichtungen (A1-A9) betrieben zu werden.

14. Vorrichtung (1) nach einem der vorhergehenden Ansprüche, wobei ein einzelner Behälter dazu konfiguriert ist, Flüssigkeiten an zwei oder mehrere Düsen in der Vielzahl von Flüssigkeitsabgabeeinrichtungen (A1-A9) zu liefern; und/oder zwei oder mehrere Behälter dazu konfiguriert sind, Flüssigkeiten an eine einzelne Düse in der Vielzahl von Flüssigkeitsabgabeeinrichtungen (A1-A9) zu liefern.

15. Verfahren (100) zum Aufzeichnen von Daten in Nukleinsäuren, unter Verwendung einer Vielzahl von Flüssigkeitsabgabeeinrichtungen (A1-A9), die eine Flüssigkeitsabgabeeinrichtung (A1), ein Tropfensammelelement (23) und mindestens zwei andere Flüssigkeitsabgabeeinrichtungen (A2, A3) umfasst, wobei das Verfahren (100) Folgendes umfasst:
- Abgeben, durch Verwendung der Flüssigkeitsabgabeeinrichtung (A1), eines Trägertropfens (10), der von der Flüssigkeitsabgabeeinrichtung (A1) in Richtung des Tropfensammelelements (23) in einer Flugbahn fliegt, die an den mindestens zwei anderen Flüssigkeitsabgabeeinrichtungen (A2, A3) vorbeiführt,
- Abgeben, durch Verwendung von mindestens zwei anderen Flüssigkeitsabgabeeinrichtungen (A2, A3), von zwei jeweiligen Tropfen in Richtung der Flugbahn des Trägertropfens (10) auf eine synchronisierte Weise, sodass der Trägertropfen (10) nacheinander mit den zwei Tropfen kollidiert, wobei jeder Tropfen der zwei Tropfen aggregiert eine Teilmenge von Komponenten aus einer Menge von Nukleinsäurekomponenten umfasst, wodurch sich die Teilmengen von Komponenten, die Daten darstellen, während des Flugs und bevor der Trägertropfen (10) auf dem Tropfensammelelement (23) landet, das den Trägertropfen (10) sammelt, in dem Trägertropfen (10) befinden, und
- Ändern, durch mindestens einen Temperaturregler (402), einer Temperatur in einer Verarbeitungskammer (60) im Laufe der Zeit, sodass eine Temperaturabfolge im Laufe der Zeit in der Verarbeitungskammer (60) erreicht wird und die Temperaturabfolge bewirkt, dass eine biochemische Reaktion zwischen den Komponenten und Bindungsreagenzien auftritt, die sich in jedem Trägertropfen (10) befinden, der in der Verarbeitungskammer (60) positioniert ist, wobei die biochemische Reaktion bewirkt, dass die Komponenten, die sich in jedem der Trägertropfen (10) befinden, assemblieren und aneinanderbinden, um endgültige Nukleinsäuremoleküle zu bilden, die Daten darstellen,
wobei es sich bei der biochemischen Reaktion um eine Polymerase-Kettenreaktion, eine Sticky-End-Ligation, eine zellenfreie Klonierung, eine BioBricks-Assemblierung, eine Gibson-Assemblierung oder eine HiFi-Assemblierung handelt und wobei mindestens einige der biochemischen Reaktionen eine Vervielfältigung der endgültigen Nukleinsäuremoleküle beinhalten.

## Revendications

1. Dispositif (1) d'enregistrement de données dans des acides nucléiques, comprenant :
- une pluralité de distributeurs de liquide (A1-A9) qui comprend un distributeur de liquide (A1) configuré pour distribuer une goutte porteuse (10),
- un élément de collecte de goutte (23) configuré pour collecter la goutte porteuse (10), dans lequel pendant le fonctionnement du dispositif (1), la goutte porteuse (10) vole à partir du distributeur de liquide (A1) vers l'élément de collecte de goutte (23) dans une trajectoire, et
- au moins deux autres distributeurs de liquide (A2, A3) de la pluralité de distributeurs de liquide (A1-A9), disposés de sorte que la trajectoire de la goutte porteuse passe par les au moins deux autres distributeurs de liquide (A2, A3), dans lequel les au moins deux autres distributeurs de liquide (A2, A3) sont configurés pour distribuer deux gouttes respectives vers la trajectoire de la goutte porteuse (10) d'une manière synchronisée, de sorte que la goutte porteuse (10) entre en collision ultérieurement avec les deux gouttes, dans lequel chaque goutte des deux gouttes comprend de manière agrégée un sous-ensemble de composants à partir d'un ensemble de composants d'acide nucléique, ainsi les sous-ensembles de composants, représentant des données, sont situés dans la goutte porteuse (10) pendant le vol et avant que la goutte porteuse ne tombe sur l'élément de collecte de goutte (23),
dans lequel le dispositif (1) comprend une chambre de traitement (60) ayant au moins un régulateur de température (402) configuré pour modifier une température dans la chambre de traitement (60) au fil du temps de sorte qu'une séquence de températures soit atteinte au fil du temps dans la chambre de traitement (60), et la séquence de températures provoque une réaction biochimique entre les composants et les réactifs de liaison situés dans chaque goutte porteuse (10) positionnée dans la chambre de traitement (60), dans lequel la réaction biochimique provoque l'assemblage et la liaison des composants situés dans chacune des gouttes porteuses (10) pour former des molécules d'acide nucléique finales, qui représentent des données,
dans lequel la réaction biochimique est une réaction en chaîne par polymérase, une ligature d'extrémité collante, un clonage sans cellule, un assemblage de bio-briques, un assemblage de Gibson ou un assemblage HiFi, et dans lequel au moins certaines des réactions biochimiques incluent une multiplication des molécules d'acide nucléique finales.

2. Dispositif (1) selon la revendication 1, **caractérisé en ce qu'**au moins un distributeur de liquide (A1) est configuré pour distribuer un flux (33) de gouttes porteuses (10), dans lequel le flux (33) comprend une première goutte porteuse (10), une deuxième goutte porteuse (10a) et au moins une troisième goutte porteuse (10b), et la pluralité de distributeurs de liquide (A1-A9) sont configurés de sorte que la première goutte porteuse (10) entre en collision avec au moins des gouttes qui comprennent de manière agrégée des composants provenant d'un premier sous-ensemble, la deuxième goutte porteuse (10a) entre en collision avec au moins des gouttes qui comprennent de manière agrégée des composants provenant d'un deuxième sous-ensemble, la troisième goutte porteuse (10b) entre en collision avec au moins des gouttes qui comprennent de manière agrégée des composants provenant d'un troisième sous-ensemble, et ainsi de suite, jusqu'à ce que toutes les gouttes porteuses du flux (33) entrent en collision avec au moins certaines gouttes qui comprennent de manière agrégée au moins certains composants provenant de l'ensemble, et toutes les gouttes porteuses (10, 10a, 10b) du flux (33) tombent sur l'élément de collecte de goutte (23).

3. Dispositif (1) selon l'une quelconque des revendications précédentes, **caractérisé en ce que** les deux ou plusieurs distributeurs de liquide (A2-A5, A6-A9) dans la pluralité de distributeurs de liquide (A1-A9) sont agencés selon un réseau (11-14), tel qu'un réseau linéaire ou un réseau circulaire.

4. Dispositif (1) selon la revendication 1 ou 2, dans lequel au moins certains des distributeurs de liquide sont agencés dans au moins un premier réseau (11-14) et un deuxième réseau (11-14), dans lequel le premier réseau (11-14) et le deuxième réseau (11-14) sont configurés pour distribuer des gouttes vers un seul flux (33) des gouttes porteuses (10, 10a, 10b), ou le premier réseau (11-14) est configuré pour distribuer des gouttes vers un premier flux (33) de gouttes porteuses (10, 10a, 10b), et le deuxième réseau (11-14) est configuré pour distribuer des gouttes vers un deuxième flux (33) de gouttes porteuses (10, 10a, 10b).

5. Dispositif (1) selon l'une quelconque des revendications précédentes, **caractérisé en ce que** chacun des distributeurs de liquide dans la pluralité de distributeurs de liquide (A1-A9) est configuré pour distribuer jusqu'à cent mille (par exemple 100 000) gouttes par seconde, et/ou est configuré pour distribuer des gouttes d'un volume dans une plage entre un picolitre (par exemple 1 pl) et cent nanolitres (par exemple 100 nl) ; et/ou la pluralité de distributeurs de liquide (A1-A9) comprend cinquante ou plus, ou cinq cents ou plus, ou dix mille ou plus de distributeurs de liquide.

6. Dispositif (1) selon l'une quelconque des revendications précédentes, **caractérisé en ce que** l'élément de collecte de goutte (23) est constitué d'un matériau pliable ou d'un matériau non pliable, et/ou est détachable ou non détachable par rapport au dispositif (1).

7. Dispositif (1) selon l'une quelconque des revendications précédentes, dans lequel l'élément de collecte de goutte (23) est un récipient rempli d'un liquide dans lequel les gouttes porteuses (10) tombent, et/ou est une surface.

8. Dispositif (1) selon l'une quelconque des revendications précédentes, **caractérisé en ce que** la pluralité de distributeurs de liquide (A1-A9) sont configurés pour distribuer des gouttes d'un premier liquide, et l'élément de collecte de goutte (23) est rempli ou recouvert d'un deuxième liquide, et le premier liquide et le deuxième liquide sont immiscibles.

9. Dispositif (1) selon l'une quelconque des revendications précédentes, **caractérisé en ce que** le dispositif (1) comprend des plaques électriques (21) agencées perpendiculairement au trajet d'au moins une goutte distribuée à partir d'au moins un distributeur de liquide (A1), dans lequel les plaques électriques (21) comportant des trous à travers lesquels passe l'au moins une goutte, l'au moins un distributeur de liquide (A1) est configuré pour fournir une charge électrique à l'au moins une goutte, et les plaques électriques (21) sont configurées de sorte que les plaques électriques (21) exercent des champs électriques sur l'au moins une goutte de manière synchronisée, de sorte que l'au moins une goutte soit manipulée avec les champs électriques exercés par les plaques électriques (21) pendant le temps pendant lequel l'au moins une goutte traverse les champs électriques exercés par les plaques électriques (21).

10. Dispositif (1) selon l'une quelconque des revendications précédentes, **caractérisé en ce que** le dispositif (1) comprend une chambre de mélange (50), qui est un compartiment partiellement ou totalement fermé du dispositif (1) dans lequel sont positionnés la pluralité de distributeurs de liquide (A1-A9) et au moins un élément de collecte de goutte (23), et la chambre de mélange (50) est le compartiment totalement fermé durant le temps pendant lequel les gouttes sont en vol.

11. Dispositif (1) selon l'une quelconque des revendications précédentes, **caractérisé en ce que** la goutte porteuse (10) inclut des réactifs de liaison, qui permettent un assemblage et une liaison des composants situés dans la goutte porteuse (10), dans lequel les réactifs de liaison sont inclus dans la goutte porteuse (10) ou fournis par une goutte qui entre en collision et fusionne avec la goutte porteuse (10) pendant le vol.

12. Dispositif (1) selon l'une quelconque des revendications précédentes, **caractérisé en ce que** le dispositif (1) comprend une mécanisation (22) configurée pour effectuer l'une quelconque ou une combinaison quelconque des étapes consistant à :
- déplacer l'élément de collecte de goutte (23) dans au moins une direction par rapport à la pluralité de distributeurs de liquide (A1-A9),
- déplacer la pluralité de distributeurs de liquide (A1-A9) dans au moins une direction par rapport à l'élément de collecte de goutte (23),
- déplacer l'élément de collecte de goutte (23) à partir de la chambre de mélange (50) vers la chambre de traitement (60).

13. Dispositif (1) selon l'une quelconque des revendications précédentes, **caractérisé en ce que** chacun des distributeurs de liquide dans la pluralité de distributeurs de liquide (A1-A9) comprend au moins un récipient destiné à contenir des liquides à distribuer, un élément générateur de goutte positionné et configuré pour provoquer la distribution d'au moins une goutte, une buse à travers laquelle l'au moins une goutte est éjectée, un capillaire qui relie le récipient à la buse de sorte que le liquide s'écoule à partir du récipient à travers le capillaire et sort à travers la buse pour que l'au moins une goutte soit distribuée, et des moyens de synchronisation, qui permettent à chacun des distributeurs de liquide de fonctionner de manière synchronisée avec d'autres distributeurs de liquide dans la pluralité de distributeurs de liquide (A1-A9).

14. Dispositif (1) selon l'une quelconque des revendications précédentes, dans lequel un seul récipient est configuré pour alimenter en liquide deux ou plusieurs buses dans la pluralité de distributeurs de liquide (A1-A9) ; et/ou deux ou plusieurs récipients sont configurés pour alimenter en liquide une seule buse dans la pluralité de distributeurs de liquide (A1-A9).

15. Procédé (100) d'enregistrement de données dans des acides nucléiques, utilisant une pluralité de distributeurs de liquide (A1-A9) qui comprennent un distributeur de liquide (A1), un élément de collecte de goutte (23) et au moins deux autres distributeurs de liquide (A2, A3), le procédé (100) comprenant :
- la distribution, en utilisant le distributeur de liquide (A1), d'une goutte porteuse (10) qui vole à partir du distributeur de liquide (A1) vers l'élément de collecte de goutte (23) dans une trajectoire qui passe par l'au moins deux autres distributeurs de liquide (A2, A3),
- la distribution, en utilisant au moins deux autres distributeurs de liquide (A2, A3), de deux gouttes respectives vers la trajectoire de la goutte porteuse (10) d'une manière synchronisée, de sorte que la goutte porteuse (10) entre en collision ultérieurement avec les deux gouttes, dans lequel chaque goutte des deux gouttes comprend de manière agrégée un sous-ensemble de composants à partir d'un ensemble de composants d'acide nucléique, ainsi les sous-ensembles de composants, représentant des données, sont situés dans la goutte porteuse (10) pendant le vol et avant que la goutte porteuse (10) ne tombe sur l'élément de collecte de goutte (23), qui collecte la goutte porteuse (10), et
- la modification, par au moins un régulateur de température (402), d'une température dans une chambre de traitement (60) au fil du temps de sorte qu'une séquence de températures soit atteinte au fil du temps dans la chambre de traitement (60), et la séquence de températures provoque une réaction biochimique entre les composants et les réactifs de liaison situés dans chaque goutte porteuse (10) positionnée dans la chambre de traitement (60), dans lequel la réaction biochimique provoque l'assemblage et la liaison des composants situés dans chacune des gouttes porteuses (10) pour former des molécules d'acide nucléique finales, qui représentent des données,
dans lequel la réaction biochimique est une réaction en chaîne par polymérase, une ligature d'extrémité collante, un clonage sans cellule, un assemblage de bio-briques, un assemblage de Gibson ou un assemblage HiFi, et dans lequel au moins certaines des réactions biochimiques incluent une multiplication des molécules d'acide nucléique finales.
